(19) Europäisches Patentamt / European Patent Office / Office européen des brevets

(11) **EP 4 678 117 A1**

(12) **EUROPEAN PATENT APPLICATION**
published in accordance with Art. 153(4) EPC

(43) Date of publication:
**14.01.2026  Bulletin 2026/03**

(21) Application number: **24770298.8**

(22) Date of filing: **06.02.2024**

(51) International Patent Classification (IPC):
$A61B\ 8/13^{(2006.01)}$  $G01N\ 29/06^{(2006.01)}$
$G01S\ 7/02^{(2006.01)}$  $G01S\ 13/89^{(2006.01)}$
$G01S\ 15/89^{(2006.01)}$

(52) Cooperative Patent Classification (CPC):
A61B 8/13; G01N 29/06; G01S 7/02; G01S 13/89;
G01S 15/89

(86) International application number:
**PCT/JP2024/003831**

(87) International publication number:
**WO 2024/190178 (19.09.2024 Gazette 2024/38)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **10.03.2023  JP 2023037702**

(71) Applicant: **K-Theory Inc.**
**Kobe-shi, Hyogo 651-0087 (JP)**

(72) Inventors:
• **KIMURA, Fumitoshi**
**Kobe-shi, Hyogo 651-0087 (JP)**
• **KIMURA, Kenjiro**
**Kobe-shi, Hyogo 651-0087 (JP)**
• **KIMURA, Noriaki**
**Kobe-shi, Hyogo 651-0087 (JP)**

(74) Representative: **KATZAROV S.A.**
**Geneva Business Center**
**12 Avenue des Morgines**
**1213 Petit-Lancy (CH)**

(54) **VISUALIZATION DEVICE AND VISUALIZATION METHOD**

(57)     An imaging device (100) includes: one or more transmitter arrays (111) each of which transmits a wave to a measurement area via a plurality of transmitters (101) that are connected in a first direction and operate in conjunction with each other; one or more receiver arrays (112) each of which receives a scattered wave of the wave from the measurement area via a plurality of receivers (102) that are connected in a second direction different from the first direction and operate in conjunction with each other; and an information processing circuit (130) that images an object in the measurement area using measurement data of the scattered wave. The information processing circuit (130) derives a scattering field function using the measurement data, derives an imaging function using the scattering field function, and images the object using the imaging function.

FIG. 11

# EP 4 678 117 A1

**Description**

[Technical Field]

The present disclosure relates to an imaging device, etc., that images an object in a measurement area using measurement data of scattered waves.

[Background Art]

As techniques related to an imaging device or the like for imaging an object in a measurement area using measurement data of scattered waves, there are techniques described in Patent Literatures (PTLs) 1 to 7.

For example, according to the technique described in PTL 1, beams sent out from a microwave sender are incident on an object to be inspected, and the amplitudes and phases of scattered beams are detected by a microwave detector. Then, the distribution of dielectric constants is calculated from output signals output from the microwave detector to display a tomogram of the object to be inspected.

[Citation List]

[Patent Literature]

[PTL 1] Japanese Unexamined Patent Application Publication No. S62-66145
[PTL 2] WO 2014/125815
[PTL 3] WO 2015/136936
[PTL 4] WO 2021/020387
[PTL 5] WO 2021/053971
[PTL 6] WO 2022/260112
[PTL 7] WO 2022/265017

[Summary of Invention]

[Technical Problem]

However, it is not easy to image an object in a measurement area using measurement data of scattered waves. More specifically, estimating measurement data on scattered waves radiated from the measurement area in relation to waves that are incident on the measurement area when the condition in the measurement area is known is called a forward problem and is easy. However, obtaining the condition in the measurement area when measurement data on scattered waves is known is called an inverse problem and is not easy.

Moreover, when a plurality of transmitters transmit waves in coordination with each other, and a plurality of receivers receive scattered waves of the waves in coordination with each other, the granularity of information may become coarse, making it potentially difficult to image an object in the measurement area.

In view of this, the present disclosure provides an imaging device or the like that is capable of efficiently imaging an object in a measurement area using a plurality of transmitters that transmit waves in coordination with each other, and a plurality of receivers that receive scattered waves of the waves in coordination with each other.

[Solution to Problem]

An imaging device according to one aspect of the present disclosure includes: one or more transmitter arrays each of which (i) includes a plurality of transmitters that are connected in a first direction and operate in conjunction with each other and (ii) transmits a wave to a measurement area via the plurality of transmitters; one or more receiver arrays each of which (i) includes a plurality of receivers that are connected in a second direction different from the first direction and operate in conjunction with each other and (ii) receives a scattered wave of the wave from the measurement area via the plurality of receivers; and an information processing circuit that images an object in the measurement area using measurement data of the scattered wave, wherein in imaging the object, the information processing circuit: derives, using the measurement data, a scattering field function that receives a transmission position of the wave and a reception position of the scattered wave as input and outputs an amount of the scattered wave at the reception position; derives an imaging function that receives an imaging target position as input and outputs an image intensity at the imaging target position, and is defined using an amount output from the scattering field function in response to inputting the imaging target position into the scattering field function as the transmission position and the reception position; and images the object in the measurement area using the imaging function.

These general or specific aspects may be implemented as a system, a device or apparatus, a method, an integrated circuit, a computer program, or a non-transitory computer-readable recording medium such as a CD-ROM, or any combination thereof.

[Advantageous Effects of Invention]

According to the present disclosure, it is possible to efficiently image an object in a measurement area using a plurality of transmitters that transmit waves in coordination with each other, and a plurality of receivers that receive scattered waves of the waves in coordination with each other.

[Brief Description of Drawings]

[FIG. 1]

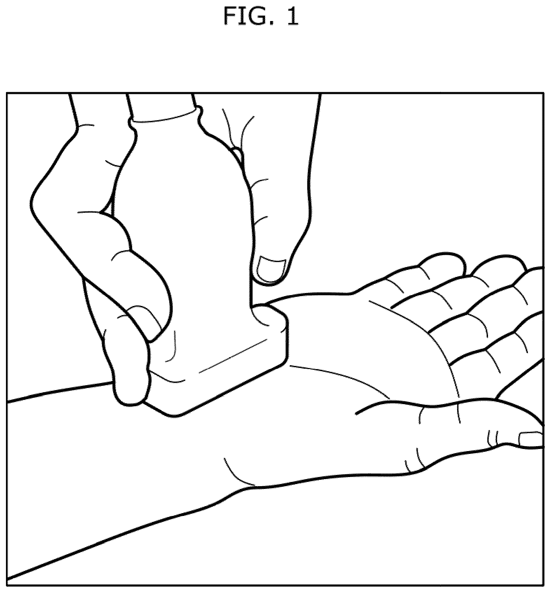

FIG. 1 is an external view illustrating an ultrasonic transmission and reception device according to a reference example.
[FIG. 2]

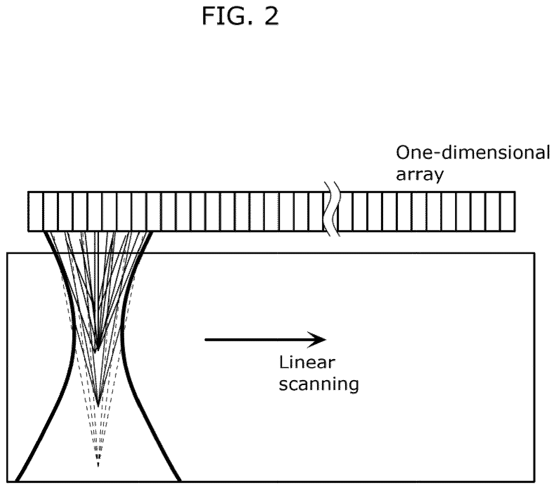

FIG. 2 is a conceptual diagram illustrating an ultrasonic dynamic depth focus according to a reference example.
[FIG. 3]

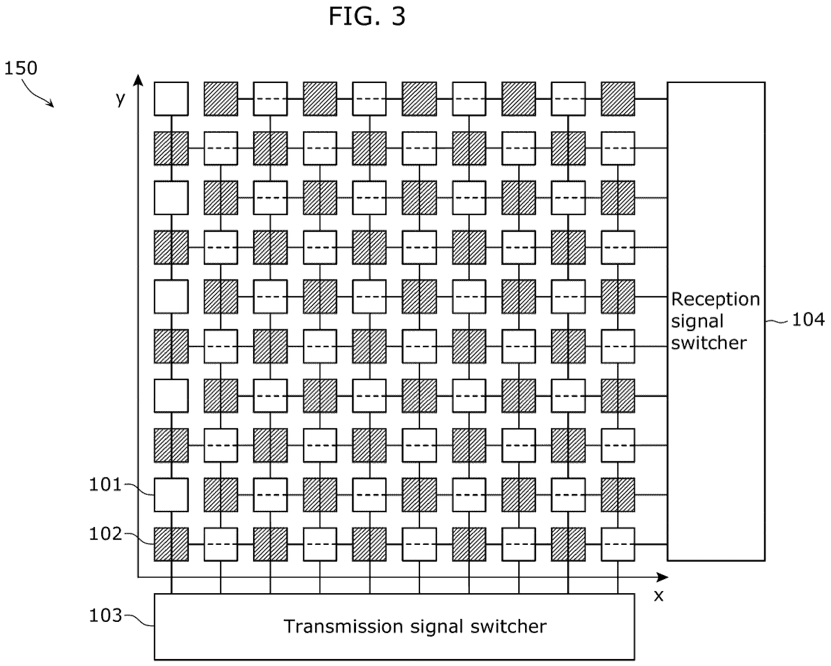

FIG. 3 is a conceptual diagram illustrating a configuration example of a two-dimensional transceiver element array according to the embodiment.
[FIG. 4]

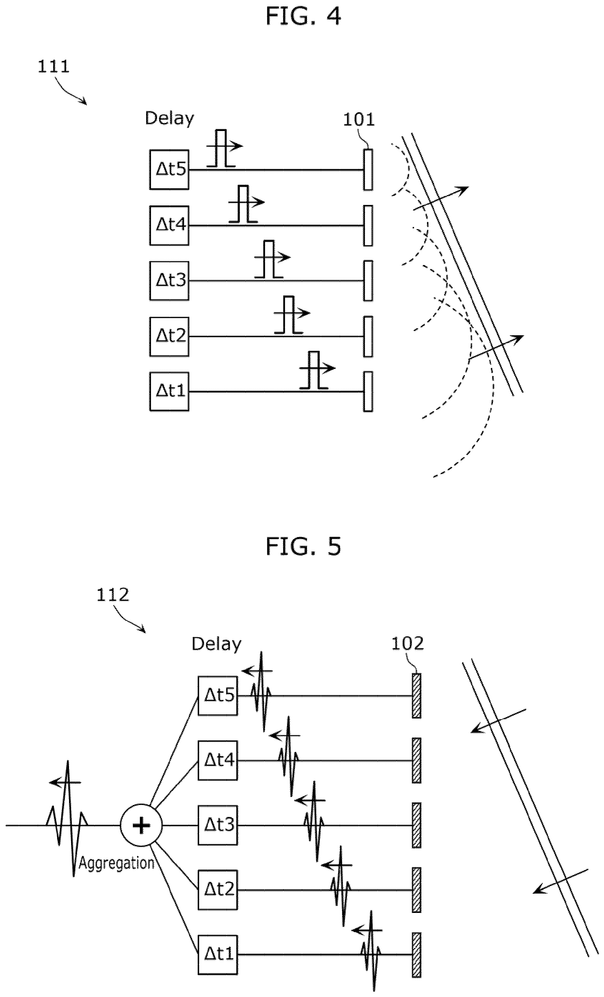

FIG. 4 is a conceptual diagram illustrating an operation example of a transmitter array.
[FIG. 5]
FIG. 5 is a conceptual diagram illustrating an operation example of a receiver array.
[FIG. 6]

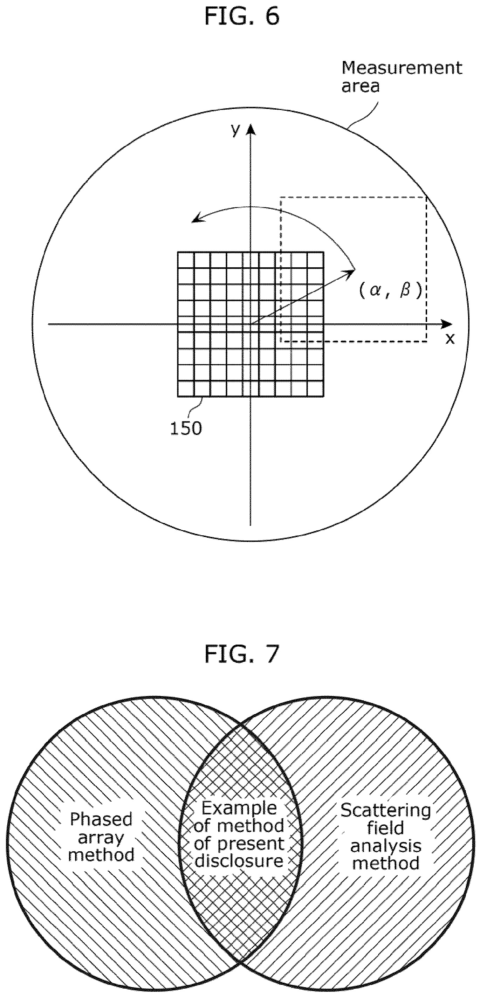

FIG. 6 is a conceptual diagram illustrating a measurement area.
[FIG. 7]
FIG. 7 illustrates the relationship between a phased array method and a scattering field analysis method.
[FIG. 8]

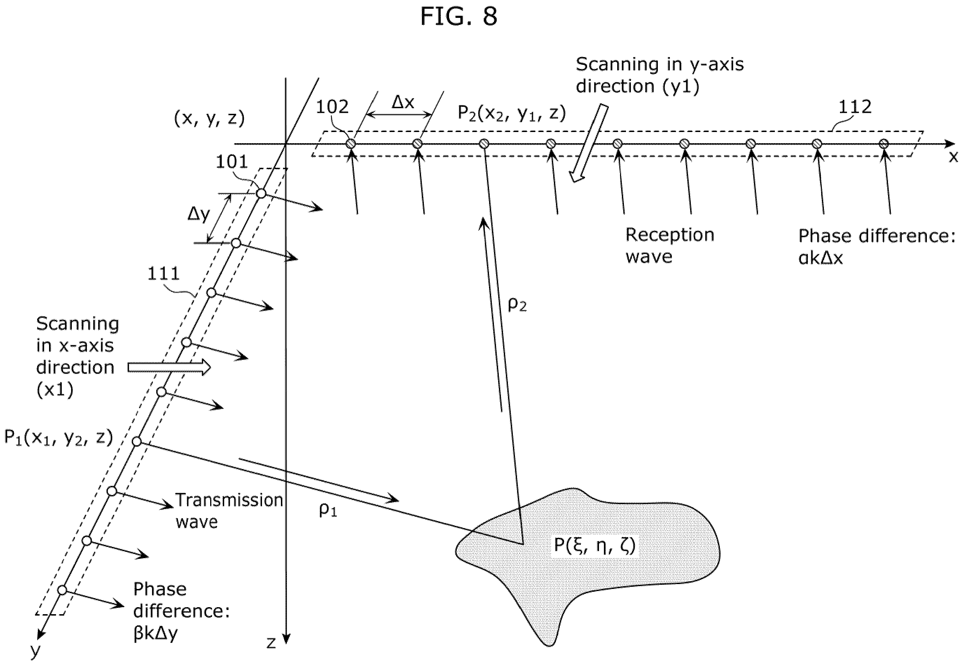

FIG. 8 is a schematic diagram illustrating the positions of a transmitter array and a receiver array.
[FIG. 9]

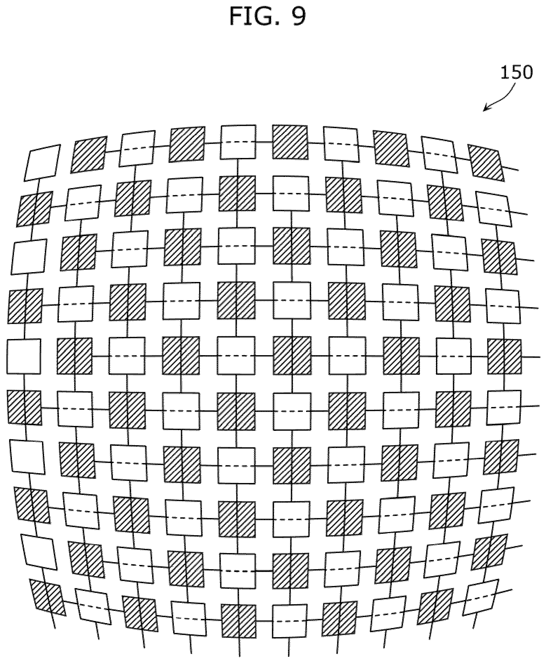

FIG. 9 is a conceptual diagram illustrating a two-dimensional transceiver element array arranged on a curved surface.
[FIG. 10]

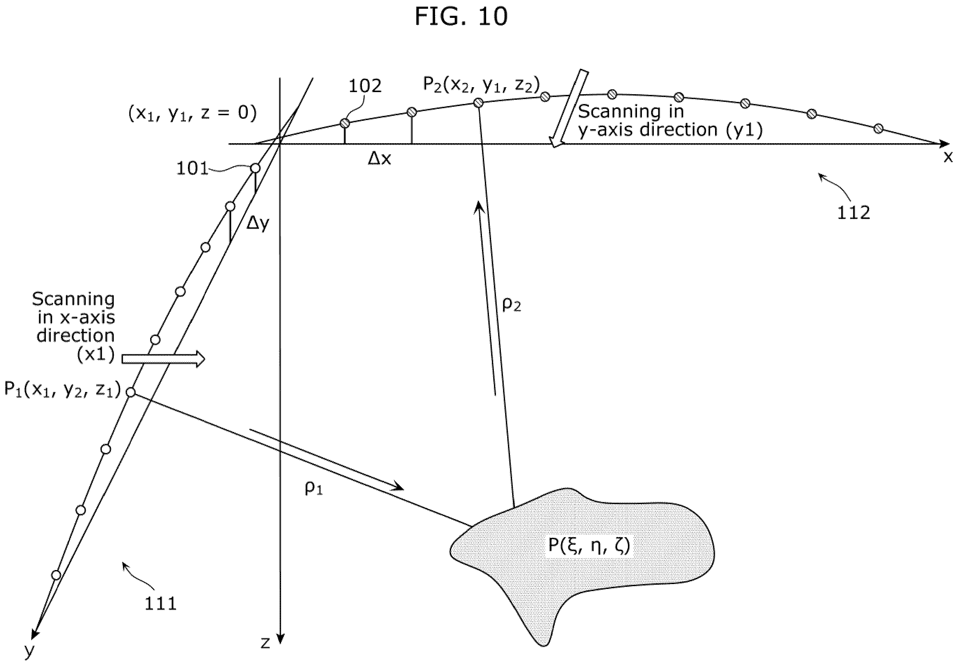

FIG. 10 is a schematic diagram illustrating positions of a transmitter array and a receiver array in a two-dimensional transceiver element array arranged on a curved surface.
[FIG. 11]

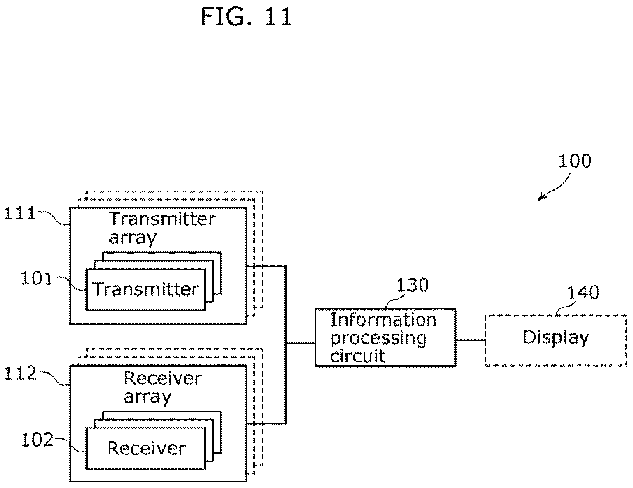

FIG. 11 is a block diagram showing a basic configuration of an imaging device.
[FIG. 12]

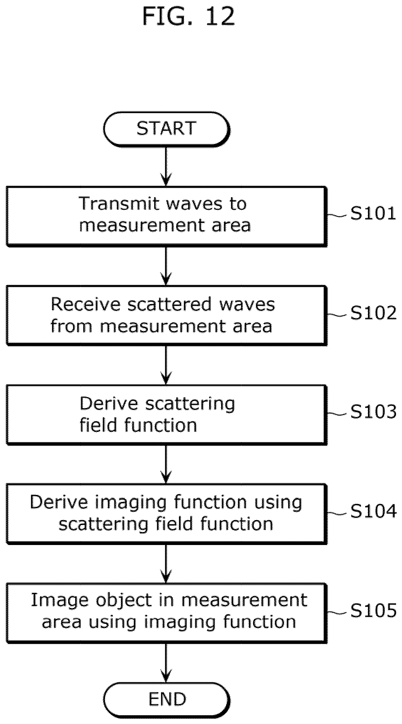

FIG. 12 is a flowchart showing basic operations of an imaging device.

[Description of Embodiments]

An imaging device according to one aspect of the present disclosure includes: one or more transmitter arrays each of which (i) includes a plurality of transmitters that are connected in a first direction and operate in conjunction with each other and (ii) transmits a wave to a measurement area via the plurality of transmitters; one or more receiver arrays each of which (i) includes a plurality of receivers that are connected in a second direction different from the first direction and operate in conjunction with each other and (ii) receives a scattered wave of the wave from the measurement area via the plurality of receivers; and an information processing circuit that images an object in the measurement area using measurement data of the scattered wave, wherein in imaging the object, the information processing circuit: derives, using the measurement data, a scattering field function that receives a transmission position of the wave and a reception position of the scattered wave as input and outputs an amount of the scattered wave at the reception position; derives an imaging function that receives an imaging target position as input and outputs an image intensity at the imaging target position, and is defined using an amount output from the scattering field function in response to inputting the imaging target position into the scattering field function as the transmission position and the reception position; and images the object in the measurement area using the imaging function.

This makes it possible to obtain measurement data corresponding to two directions using a plurality of transmitters that transmit waves in coordination with each other, and a plurality of receivers that receive scattered waves of the waves in coordination with each other. This makes it possible to accurately derive the scattering field function and the imaging function using the measurement data corresponding to two directions. This in turn makes it possible to image the object in the measurement area efficiently.

For example, each of the one or more transmitter arrays and the one or more receiver arrays is a phased array.

This makes it possible to transmit a wave in an adaptively determined direction and to receive a scattered wave from an adaptively determined direction. This in turn makes it possible to efficiently image the object in an adaptively determined range.

For example, the one or more transmitter arrays is one transmitter array that moves in the second direction, and the one or more receiver arrays is one receiver array that moves in the first direction.

This makes it possible to reduce the plurality of transmitter arrays and the plurality of receiver arrays. Therefore, it becomes possible to reduce the cost for preparing and arranging the plurality of transmitter arrays and the plurality of receiver arrays.

For example, the one or more transmitter arrays includes a plurality of transmitter arrays arranged in the second direction, the one or more receiver arrays includes a plurality of receiver arrays arranged in the first direction, and the plurality of transmitter arrays sequentially transmit the wave.

This makes it possible to reduce the mechanism for moving the transmitter array and the receiver array. It also becomes possible to reduce the time required for moving the transmitter array and the receiver array during measurement.

For example, the one or more transmitter arrays and the one or more receiver arrays are arranged on a flat surface.

This makes it possible to simplify the arrangement of the one or more transmitter arrays and the one or more receiver arrays. Therefore, it becomes possible to reduce the cost for arranging the one or more transmitter arrays and the one or more receiver arrays. It also becomes possible to reduce the amount of computation required for deriving the scattering field function and imaging function.

For example, the one or more transmitter arrays and the one or more receiver arrays are arranged on a curved surface.

This makes it possible to arrange the one or more transmitter arrays and the one or more receiver arrays on a curved surface according to the characteristics of the one or more transmitter arrays, the characteristics of the one or more receiver arrays, or the characteristics of the measurement area. This makes it possible to flexibly arrange the one or more transmitter arrays and the one or more receiver arrays.

For example, the one or more transmitter arrays and the one or more receiver arrays are arranged on a flat surface, the first direction is a $\gamma$-axis direction, the second direction is an x-axis direction, the scattering field function is expressed as:

[Math. 1]

$$\Phi(x_1, y_1, z, \alpha, \beta, k) = \frac{1}{(2\pi)^2} \int_{-\infty}^{\infty} \int_{-\infty}^{\infty} e^{-i(k_{x_1}x_1 + k_{y_1}y_1)} \tilde{Q}(k_{x_1}, k_{y_1}, \alpha, \beta, k)$$

$$\cdot e^{iz\left\{\sqrt{k^2 - \left\{k_{x_1}^2 + \beta^2 k^2\right\}} \pm \sqrt{k^2 - \left\{k_{y_1}^2 + \alpha^2 k^2\right\}}\right\}} dk_{x_1} dk_{y_1}$$

where z of the scattering field function represents a z-coordinate of the transmission position and the reception position, $x_1$ of the scattering field function represents an x-coordinate of the transmission position, $y_1$ of the scattering field function represents a y-coordinate of the reception position, k represents a wavenumber of the wave, $k_{x_1}$ and $k_{y_1}$ represent wavenumbers with respect to $x_1$ and $y_1$ of the scattering field function, $\alpha$ represents a phase factor of the one or more receiver arrays, and $\beta$ represents a phase factor of the one or more transmitter arrays, and

[Math. 2]

$$\tilde{Q}$$

represents the measurement data that has been Fourier transformed.

This makes it possible to accurately derive the scattering field function according to the arrangement of the one or more transmitter arrays and the one or more receiver arrays on a flat surface. Therefore, it becomes possible to accurately derive the scattering field function corresponding to the arrangement of the one or more transmitter arrays and the one or more receiver arrays.

For example, the imaging function is expressed as:

[Math. 3]

$$\rho(x, y, z)$$

$$= \frac{1}{(2\pi)^2} \int_0^\infty \int_{-\infty}^\infty \int_{-\infty}^\infty e^{-i(k_{x_1}x + k_{y_1}y)} \sum_\alpha \sum_\beta \left\{ \tilde{Q}(k_{x_1}, k_{y_1}, \alpha, \beta, k) \cdot e^{ik_z z} \right\} dk \, dk_{x_1} dk_{y_1}$$

where x, y, and z of the imaging function respectively represent an x-coordinate, a y-coordinate, and a z-coordinate of the imaging target position, and $k_z$ is defined as:

[Math. 4]

$$k_z = \sqrt{k^2 - \left\{ k_{x_1}^2 + \beta^2 k^2 \right\}} \pm \sqrt{k^2 - \left\{ k_{y_1}^2 + \alpha^2 k^2 \right\}}$$

.

This makes it possible to accurately derive the imaging function corresponding to the scattering field function formulated according to the arrangement of the one or more transmitter arrays and the one or more receiver arrays on a flat surface.

For example, the imaging function is expressed as:

[Math. 5]

$$\rho(x, y, z)$$

$$= \frac{1}{(2\pi)^2} \int_0^\infty \int_{-\infty}^\infty \int_{-\infty}^\infty e^{-i(k_{x_1}x + k_{y_1}y)} \sum_\alpha \sum_\beta \left\{ \tilde{Q}(k_{x_1}, k_{y_1}, \alpha, \beta, k) \cdot e^{ik_z z} \right\} \left( \frac{dk}{dk_z} \right) dk_{x_1} dk_{y_1} dk_z$$

where x, y, and z of the imaging function respectively represent an x-coordinate, a y-coordinate, and a z-coordinate of the imaging target position, and $k_z$ and $dk/dk_z$ are defined as:

[Math. 6]

$$k_z = \sqrt{k^2 - \left\{ k_{x_1}^2 + \beta^2 k^2 \right\}} \pm \sqrt{k^2 - \left\{ k_{y_1}^2 + \alpha^2 k^2 \right\}}$$

$$\frac{dk}{dk_z} = \frac{1}{\frac{(1-\beta^2)k}{\sqrt{(1-\beta^2)k^2 - k_{x_1}^2}} + \frac{(1-\alpha^2)k}{\sqrt{(1-\alpha^2)k^2 - k_{y_1}^2}}}$$

.

This makes it possible to accurately derive the imaging function corresponding to the scattering field function formulated according to the arrangement of the one or more transmitter arrays and the one or more receiver arrays on a flat surface. This also makes it possible to perform calculations efficiently according to the transformed integration variables.

For example, the one or more transmitter arrays and the one or more receiver arrays are arranged on a curved surface, the first direction is a $\gamma$-axis direction, the second direction is an x-axis direction, the scattering field function is expressed as:

[Math. 7]

$$\Phi(x_1, y_1, z, k)$$

$$= \frac{1}{(2\pi)^4} \int_{-\infty}^{\infty} \int_{-\infty}^{\infty} \int_{-\infty}^{\infty} \int_{-\infty}^{\infty} e^{-i(k_{x_1} x_1 + k_{y_1} y_1)} \left\{ \sum_{x_2, y_2} e^{-i(k_{x_2} x_2 + k_{y_2} y_2)} e^{i v_2 \sqrt{k^2 - k_{x_1}^2 - k_{y_2}^2}} e^{i u_2 \sqrt{k^2 - k_{y_1}^2 - k_{x_2}^2}} \right\}$$

$$\cdot b(k_{x_1}, k_{y_1}, k) e^{iz\left(\sqrt{k^2 - k_{x_1}^2 - k_{y_2}^2} + \sqrt{k^2 - k_{y_1}^2 - k_{x_2}^2}\right)} dk_{x_1} dk_{y_1} dk_{x_2} dk_{y_2}$$

where z of the scattering field function represents a z-coordinate of the transmission position and the reception position, $x_1$ of the scattering field function represents an x-coordinate of the transmission position, $y_1$ of the scattering field function represents a y-coordinate of the reception position, k represents a wavenumber of the wave, and $k_{x1}$, $k_{y1}$, $k_{x2}$, and $k_{y2}$ represent integration variables, b is defined as:

[Math. 8]

$$b(k_{x_1}, k_{y_1}, k) = \sum_{I, J} b_{I, J}(k_{x_1}, k_{y_1}, k)$$

where I represents an index of an x-coordinate at which the one or more transmitter arrays transmit the wave, and J represents an index of a y-coordinate at which the one or more receiver arrays receive the scattered wave, $b_{I,J}$ is defined as:

[Math. 9]

$$b_{I, J}(k_{x_1}, k_{y_1}, k) = \frac{e^{i(k_{x_1} x_I + k_{y_1} y_J)} \Phi(x_I, y_J, 0, k)}{\frac{1}{(2\pi)^2} \int_{-\infty}^{\infty} \int_{-\infty}^{\infty} G_{I, J}(k_{x_1}, k_{y_1}, k_{x_2}, k_{y_2}, k) dk_{x_2} dk_{y_2}}$$

where $\Phi(x_I, y_J, 0, k)$ represents the measurement data, and $G_{I,J}$ is defined as:

[Math. 10]

$$G_{I, J}(k_{x_1}, k_{y_1}, k_{x_2}, k_{y_2}, k)$$

$$= \sum_{m=-\infty}^{\infty} \sum_{n=-\infty}^{\infty} e^{-i(k_{x_2} m\Delta x + k_{y_2} n\Delta y)} e^{i v_2(x_I, n\Delta y) \sqrt{k^2 - k_{x_1}^2 - k_{y_2}^2}} e^{i u_2(y_J, m\Delta x) \sqrt{k^2 - k_{y_1}^2 - k_{x_2}^2}}$$

where $\Delta x$ represents a distance between two adjacent receivers among the plurality of receivers, $\Delta y$ represents a distance between two adjacent transmitters among the plurality of transmitters, and each of $v_2$ and $u_2$ represents a function expressing a shape of the curved surface.

This makes it possible to accurately derive the scattering field function according to the arrangement of the one or more transmitter arrays and the one or more receiver arrays on a curved surface. Therefore, it becomes possible to accurately derive the scattering field function corresponding to the arrangement of the one or more transmitter arrays and the one or more receiver arrays.

For example, the imaging function is expressed as:

$$[\text{Math. 11}]$$

$$\rho(x, y, z) = \int_0^\infty \Phi(x, y, z, k)\, dk$$

where x, y, and z of the imaging function respectively represent an x-coordinate, a y-coordinate, and a z-coordinate of the imaging target position.

This makes it possible to accurately derive the imaging function corresponding to the scattering field function formulated according to the arrangement of the one or more transmitter arrays and the one or more receiver arrays on a flat surface.

An imaging method according to one aspect of the present disclosure includes: transmitting a wave to a measurement area via a plurality of transmitters included in one or more transmitter arrays in each of which the plurality of transmitters are connected in a first direction and operate in conjunction with each other; receiving a scattered wave of the wave from the measurement area via a plurality of receivers included in one or more receiver arrays in each of which the plurality of receivers are connected in a second direction different from the first direction and operate in conjunction with each other; and imaging an object in the measurement area using measurement data of the scattered wave, wherein the imaging of the object includes: deriving, using the measurement data, a scattering field function that receives a transmission position of the wave and a reception position of the scattered wave as input and outputs an amount of the scattered wave at the reception position; deriving an imaging function that receives an imaging target position as input and outputs an image intensity at the imaging target position, and is defined using an amount output from the scattering field function in response to inputting the imaging target position into the scattering field function as the transmission position and the reception position; and imaging the object in the measurement area using the imaging function.

This makes it possible to obtain measurement data corresponding to two directions using a plurality of transmitters that transmit waves in coordination with each other, and a plurality of receivers that receive scattered waves of the waves in coordination with each other. This makes it possible to accurately derive the scattering field function and the imaging function using the measurement data corresponding to two directions. This in turn makes it possible to image the object in the measurement area efficiently.

Hereinafter, embodiments will be described with reference to the drawings. Each of the following embodiments describes a general or specific example. The numerical values, shapes, materials, elements, the arrangement and connection of the elements, steps, the order of the steps etc., presented in the following embodiments are mere examples, and do not limit the scope of the claims.

In the following description, in particular the techniques or the like described in PTLs 2 to 7 given above may be referred to as existing techniques. Although ultrasonic waves are primarily assumed as the waves in the following description, the waves are not limited to these, and may be millimeter waves, other elastic waves, other radio waves, or other electromagnetic waves or the like. Imaging based on scattering may be expressed as scattering tomography. Thus, the imaging device and imaging method described below may also be expressed as a scattering tomographic device and a scattering tomographic method, respectively.

[Embodiment]

An imaging device according to the present embodiment images an object in a measurement area using measurement data of scattered waves. Hereinafter, the imaging device according to the present embodiment, including techniques and theories serving as the basis of the imaging device, will be described in detail.

<1. Overview>

The present disclosure describes a technique for generating real-time three-dimensional images primarily using ultrasound. However, similar transmission and reception methods and image reconstruction methods can be applied to other waves that are physically different from ultrasound, such as millimeter waves.

Ultrasonic imaging devices have been widely used in various fields for a long time, particularly in the medical field and in the field of non-destructive testing of metal structures and the like. In the medical field, ultrasonic imaging devices are also called echo devices or ultrasonic diagnostic devices, and are used for ultrasonic diagnosis.

FIG. 1 is an external view illustrating an ultrasonic transmission and reception device according to a reference example. The ultrasonic transmission and reception device illustrated in FIG. 1 is used in medical ultrasonic imaging devices. In such ultrasonic transmission and reception devices, either a single pair of a transmitter and a receiver, or a one-dimensional array is overwhelmingly often used for the probe.

FIG. 2 is a conceptual diagram illustrating an ultrasonic dynamic depth focus according to a reference example. For example, in medical ultrasonic imaging devices, a one-dimensional array is used, and a group of ultrasonic signals that have a focus at a desired depth in space, as illustrated in FIG. 2, is used. More specifically, processing delays are applied to the ultrasonic signals for individual transceiver elements to maintain focus at a certain depth. The focus depth may be controlled in one stage or two stages. Furthermore, by performing linear scanning on the one-dimensional array, a B-mode image for ultrasound diagnosis is obtained.

However, if there is an object that reflects ultrasonic waves in front of the focus position, it is difficult to obtain information at the focus position. Obtaining a three-dimensional image is therefore not easy.

FIG. 3 is a conceptual diagram illustrating a configuration example of the two-dimensional transceiver element array according to the present embodiment. Two-dimensional transceiver element array 150 illustrated in FIG. 3 includes a plurality of transmitters 101 and a plurality of receivers 102, and is connected to transmission signal switcher 103 and reception signal switcher 104. Two-dimensional transceiver element array 150 may be, for example, a two-dimensional piezoelectric element array. Two-dimensional transceiver element array 150 may include transmission signal switcher 103 and reception signal switcher 104. The plurality of elements illustrated in FIG. 3 may be included in the imaging device.

Transmitter 101 is a piezoelectric element that transmits waves such as ultrasonic waves, and is shown as a square without hatching in FIG. 3. The plurality of transmitters 101 constitute a plurality of transmitter arrays, each of which is a one-dimensional array. Each transmitter array includes a plurality of transmitters 101 connected in the $\gamma$-axis direction, and is connected to transmission signal switcher 103. The plurality of transmitters 101 included in each transmitter array transmit waves in coordination with each other.

Receiver 102 is a piezoelectric element that receives waves such as ultrasonic waves, and is shown as a square with hatching in FIG. 3. The plurality of receivers 102 constitute a plurality of receiver arrays, each of which is a one-dimensional array. Each receiver array includes a plurality of receivers 102 connected in the x-axis direction, and is connected to reception signal switcher 104. The plurality of receivers 102 included in each receiver array receive waves in coordination with each other.

Transmission signal switcher 103 is a circuit that controls wave transmission from each transmitter array. Reception signal switcher 104 is a circuit that controls wave reception from each receiver array. By connecting to transmitters 101 located at the end of the transmitter arrays, transmission signal switcher 103 indirectly connects to other transmitters 101 included in the transmitter arrays. Similarly, by connecting to receivers 102 located at the end of the receiver arrays, reception signal switcher 104 indirectly connects to other receivers 102 included in the receiver arrays. This simplifies the wiring and control.

In the present embodiment, the direction in which the plurality of transmitters 101 are connected differs from the direction in which the plurality of receivers 102 are connected. Stated differently, the direction in which the transmitter arrays are arranged differs from the direction in which the receiver arrays are arranged. This makes it possible to obtain information corresponding to a plurality of directions.

More specifically, the direction in which the transmitter arrays are arranged is orthogonal to the direction in which the receiver arrays are arranged. Stated differently, the plurality of transmitters 101 and the plurality of receivers 102 constitute an orthogonal two-dimensional array. This simplifies the processing of information obtained by the plurality of transmitters 101 and the plurality of receivers 102.

Furthermore, in the present embodiment, the transmitter array and the receiver array may operate as a phased array.

FIG. 4 is a conceptual diagram illustrating an operation example of a transmitter array. In FIG. 4, the plurality of transmitters 101 constitute transmitter array 111. The plurality of transmitters 101 are connected in the y-axis direction as illustrated in FIG. 3. Transmitter array 111 may include a plurality of delay devices respectively corresponding to the plurality of transmitters 101.

By each transmitter 101 transmitting a wave, transmitter array 111 is capable of transmitting a single composite wave formed by combining the plurality of waves. Transmitter array 111 is capable of controlling the direction of the transmitted wave by controlling the delay amount of the transmission process ($\Delta t_1$ to $\Delta t_5$ in FIG. 4). This allows transmitter array 111 to operate as a phased array and transmit a wave in a desired direction.

The control of the delay is not limited to being performed by transmitter array 111, and may be performed by transmitter 101, or may be performed by other elements.

FIG. 5 is a conceptual diagram illustrating an operation example of a receiver array. In FIG. 5, the plurality of receivers 102 constitute receiver array 112. The plurality of receivers 102 are connected in the x-axis direction as illustrated in FIG. 3. Receiver array 112 may include a plurality of delay devices respectively corresponding to the plurality of receivers 102. Receiver array 112 may further include an aggregator device that aggregates wave signals.

By each receiver 102 receiving a wave, receiver array 112 is capable of receiving a single composite wave formed by combining the plurality of waves. Receiver array 112 is capable of controlling the direction of the received wave by controlling the delay amount of the reception process ($\Delta t_1$ to $\Delta t_5$ in FIG. 5). This allows receiver array 112 to operate as a phased array and receive a wave from a desired direction.

The control of the delay and compositing is not limited to being performed by receiver array 112, and may be performed by receiver 102, or may be performed by other elements.

FIG. 6 is a conceptual diagram illustrating a measurement area. More specifically, FIG. 6 illustrates a measurement area at a position separated in the z direction from two-dimensional transceiver element array 150 arranged on the xy-plane.

The imaging device according to the present embodiment transmits a wave to the measurement area and receives scattered waves of the wave from the measurement area. The imaging device images an object in a measurement area using measurement data of scattered waves. Accordingly, if it is possible to transmit a wave to a distant location and receive scattered waves from a distant location, it is possible to image a distant object.

The plurality of transmitters 101 in two-dimensional transceiver element array 150 can efficiently transmit waves to distant locations by transmitting waves in coordination with each other. The plurality of receivers 102 in two-dimensional transceiver element array 150 can efficiently receive scattered waves from distant locations by receiving waves in coordination with each other.

Furthermore, each row and column of two-dimensional transceiver element array 150 can control the direction of transmission and the direction of reception by operating as a phased array. For example, in FIG. 6, the direction of transmission and the direction of reception are controlled by phase parameters ($\alpha$, $\beta$) determined based on the delay amounts in FIG. 4 and FIG. 5.

According to the above operation, it becomes possible to expand the measurement area at a position separated in the z direction from two-dimensional transceiver element array 150 as illustrated in FIG. 6. Furthermore, two-dimensional transceiver element array 150 makes it possible to collect information corresponding to various directions. Therefore, two-dimensional transceiver element array 150 makes it possible to generate an image having a high S/N ratio.

This enables the reconstruction of a high-precision three-dimensional image using the measurement data obtained by two-dimensional transceiver element array 150 and a scattering field analysis method.

FIG. 7 is a conceptual diagram illustrating the relationship between a phased array method and a scattering field analysis method. An example of the method of the present disclosure corresponds to a combination of the phased array method and the scattering field analysis method. The present disclosure introduces a technique that is the first of its kind in the world. An example of the method of the present disclosure can be easily implemented using ultrasound, but can also be implemented using microwaves and millimeter waves, etc.

<2. Imaging Device Using Two-dimensional Transceiver Element Array on Flat Surface>

The imaging device according to the present embodiment generates, for example, real-time three-dimensional images using ultrasound. The imaging device may be a device known as an ultrasonic imaging device. In this chapter, the imaging device uses a two-dimensional transceiver element array 150 on a flat surface.

<2-1. Two-dimensional Transceiver Element Array>

As illustrated in FIG. 6, a plurality of transmitters 101 or a plurality of receivers 102 in two-dimensional transceiver element array 150 are coupled on a column-by-column or row-by-row basis.

Transmission of waves such as ultrasonic waves is performed while switching the plurality of transmitters 101 on a column-by-column basis. The plurality of transmitters 101 within a single column may be directly connected by wires. Alternatively, the plurality of transmitters 101 within a single column may be coupled via delay elements in order to operate as a phased array.

Reception of waves such as ultrasonic waves may be performed while switching the plurality of receivers 102 on a row-by-row basis, or may be performed simultaneously in a plurality of rows of the plurality of receivers 102. The plurality of receivers 102 within a single column may be directly connected by wires. Alternatively, the plurality of receivers 102 within a single column may be coupled via delay elements in order to operate as a phased array.

For example, for transmission, one column in the two-dimensional array is selected, and for reception, one row in the two-dimensional array is selected. When the number of columns for transmission and the number of rows for reception are each N, there are $N^2$ combinations. The shape of the object is reconstructed from the scattering data corresponding to these $N^2$ combinations.

In FIG. 6, two-dimensional transceiver element array 150 includes a plurality of transmitter arrays 111 and a plurality of receiver arrays 112. However, two-dimensional transceiver element array 150 may include only one transmitter array 111 and one receiver array 112, instead of the plurality of transmitter arrays 111 and the plurality of receiver arrays 112.

One transmitter array 111 along the y-axis direction may sequentially move to a plurality of positions in the x-axis direction to transmit waves. One receiver array 112 along the x-axis direction may sequentially move to a plurality of positions in the y-axis direction to receive waves. With this, one transmitter array 111 and one receiver array 112 can fulfill the role of a plurality of transmitter arrays 111 and a plurality of receiver arrays 112.

<2-2. Scattering Field Analysis Method in Phased Array Method>

FIG. 8 is a schematic diagram illustrating the positions of transmitter array 111 and receiver array 112. Transmitter array 111 is arranged along the y-axis direction, and a plurality of transmitters 101 included in transmitter array 111 are arranged along the y-axis direction. Receiver array 112 is arranged along the x-axis direction, and a plurality of receivers 102 included in receiver array 112 are arranged along the x-axis direction.

Transmitter array 111 transmits waves from a line-shaped region extending along the y-axis direction. Transmitter array 111 performs scanning in the x-axis direction.

More specifically, transmitter array 111 may perform scanning electrically. Stated differently, a plurality of transmitter arrays 111 may be arranged in a row in the x-axis direction, and the transmitter array 111 that transmits waves may be sequentially switched in the x-axis direction from among the plurality of transmitter arrays 111. Alternatively, transmitter array 111 may perform scanning mechanically. Stated differently, one transmitter array 111 may move to a plurality of positions in the x-axis direction to transmit waves.

Receiver array 112 receives waves in a line-shaped region extending along the x-axis direction. Receiver array 112 performs scanning in the y-axis direction.

More specifically, receiver array 112 may perform scanning electrically. Stated differently, a plurality of receiver arrays 112 may be arranged in a row in the y-axis direction, and the receiver array 112 that receives waves may be sequentially switched in the y-axis direction from among the plurality of receiver arrays 112. Alternatively, receiver array 112 may perform scanning mechanically. Stated differently, one receiver array 112 may move to a plurality of positions in the y-axis direction to receive waves.

Alternatively, instead of receiver array 112 scanning, a plurality of receiver arrays 112 arranged in a row in the y-axis direction may simultaneously receive waves.

Two-dimensional transceiver element array 150 including transmitter array 111 and receiver array 112 can operate as an N×N orthogonal phased array.

Note that the x-axis direction, y-axis direction, and z-axis direction are arbitrarily defined as three mutually orthogonal directions in three-dimensional space. For example, the direction in which the plurality of receivers 102 are connected is defined as the x-axis direction. The direction perpendicular to the x-axis direction, in which the plurality of transmitters 101 are connected, is defined as the y-axis direction. The direction perpendicular to the x-axis direction and the y-axis direction is defined as the z-axis direction.

In the example of FIG. 8, a wave radiated from transmitter 101 corresponding to point $P_1(x_1, y_2, z)$ in transmitter array 111 is reflected at point $P(\xi, \eta, \zeta)$ on the object and received by receiver 102 corresponding to point $P_2(x_2, y_1, z)$ in receiver array 112. Under this condition, the scattering field equation is constructed as follows.

First, distance $\rho_1$ between point $P_1$ and point P, and distance $\rho_2$ between point $P_2$ and point P, are defined by the following expression (2-2-1).

[Math. 12]

$$\rho_1^{\ 2} = (x_1 - \xi)^2 + (y_2 - \eta)^2 + (z - \varsigma)^2$$
$$\rho_2^{\ 2} = (x_2 - \xi)^2 + (y_1 - \eta)^2 + (z - \varsigma)^2$$
$$\cdot \cdot \cdot (2-2-1)$$

Next, a function as given by expression (2-2-2) below is defined. The function of the following equation (2-2-2) corresponds to a wave transmitted from point $P_1(x_1, y_2, z)$, reflected at point $P(\xi, \eta, \zeta)$, and received at point $P_2(x_2, y_1, z)$.

[Math. 13]

$$\phi\left(x_1, y_1, x_2, y_2, z, \omega\right) = \frac{e^{ik\rho_1 + i\beta ky_2}}{\rho_1} \frac{e^{ik\rho_2 + i\alpha kx_2}}{\rho_2}$$

$$\cdot \cdot \cdot (2-2-2)$$

It is assumed here that the time factor is proportional to exp(-i$\omega$t). k is the wavenumber of the wave. $\alpha$ is a phase factor of receiver array 112, and is defined as a dimensionless value. Similarly, $\beta$ is a phase factor of transmitter array 111, and is defined as a dimensionless value. More specifically, the phase on the x-axis is defined as $akx_2$. Similarly, the phase on the $\gamma$-axis is defined as $\beta ky_2$.

In the example in FIG. 8, the distance between two adjacent receivers 102 in the x-axis direction is $\Delta x$. The distance between two adjacent transmitters 101 in the $\gamma$-axis direction is $\Delta y$. Therefore, the phase difference between two adjacent receivers 102 in the x-axis direction is $ak\Delta x$. The phase difference between two adjacent transmitters 101 in the y-axis direction is $\beta k\Delta y$.

The differentiation of function $\varphi$ is expressed as given by expression (2-2-3) below.

[Math. 14]

$$\frac{e^{ik\rho_1 + i\beta ky_2}}{\rho_1} \frac{e^{ik\rho_2 + i\alpha kx_2}}{\rho_2}$$

$$\partial_{x_1}\phi = ik\frac{(x_1 - \xi)}{\rho_1}\phi + o(\rho^{-3})$$

$$\partial_{y_1}\phi = ik\frac{y_1 - \eta}{\rho_2}\phi + o(\rho^{-3})$$

$$\partial_{x_2}\phi = ik\left(\frac{x_2 - \xi}{\rho_2} + \alpha\right)\phi + o(\rho^{-3})$$

$$\partial_{y_2}\phi = ik\left(\frac{y_2 - \eta}{\rho_1} + \beta\right)\phi + o(\rho^{-3})$$

$$\partial_z\phi = ik(z - \zeta)(\frac{1}{\rho_1} + \frac{1}{\rho_2})\phi + o(\rho^{-3})$$

$$\partial_{x_1}\partial_{x_1}\phi = (ik)^2\frac{(x_1 - \xi)^2}{\rho_1^2}\phi + o(\rho^{-3})$$

$$\partial_{x_2}\partial_{x_2}\phi = (ik)^2\left(\frac{x_2 - \xi}{\rho_2} + \alpha\right)^2\phi + o(\rho^{-3})$$

$$\partial_{y_1}\partial_{y_1}\phi = (ik)^2\frac{(y_1 - \eta)^2}{\rho_2^2}\phi + o(\rho^{-3})$$

$$\partial_{y_2}\partial_{y_2}\phi = (ik)^2\left(\frac{y_2 - \eta}{\rho_1} + \beta\right)^2\phi + o(\rho^{-3})$$

$$\partial_z\partial_z\phi = (ik)^2(z - \zeta)^2(\frac{1}{\rho_1} + \frac{1}{\rho_2})^2\phi + o(\rho^{-3})$$

$$\cdots (2-2-3)$$

Next, pseudo-differential operators $\partial_u$ and $\partial_v$ as given by expression (2-2-4) below are introduced. Symbols $k_u$ and $k_v$ corresponding to $\partial_u$ and $\partial_v$ are defined, using Fourier transform, as given on the right side of expression (2-2-4) below, respectively.
[Math. 15]

$$\partial_u = \partial_{x_2} - i\alpha k \quad : \quad k_u = k_{x_2} + \alpha k$$

$$\partial_v = \partial_{y_2} - i\beta k \quad : \quad k_v = k_{y_2} + \beta k$$

$$\cdots (2-2-4)$$

The 3rd, 4th, 7th, and 9th rows of expression (2-2-3) are expressed, using the pseudo-differential operators $\partial_u$ and $\partial_v$ of expression (2-2-4), as given by expression (2-2-5) below.
[Math. 16]

$$\partial_u \phi = ik \frac{x_2 - \xi}{\rho_2} \phi + o(\rho^{-3})$$

$$\partial_v \phi = ik \frac{y_2 - \eta}{\rho_1} \phi + o(\rho^{-3})$$

$$\partial_u \partial_u \phi = (ik)^2 \left( \frac{x_2 - \xi}{\rho_2} \right)^2 \phi + o(\rho^{-3})$$

$$\partial_v \partial_v \phi = (ik)^2 \left( \frac{y_2 - \eta}{\rho_1} \right)^2 \phi + o(\rho^{-3})$$

$$\cdots (2-2-5)$$

When the term of $O(\rho^{-3})$ is ignored, expression (2-2-6) below holds true.
[Math. 17]

$$\Delta_5 \phi = (ik)^2 \left\{ 2 + \frac{2(z - \varsigma)^2}{\rho_1 \rho_2} \right\} \phi$$

$$= (ik)^2 \left\{ 2 + 2 \sqrt{1 + \frac{1}{k^2} \partial_{x_1}^2 + \frac{1}{k^2} \partial_v^2} \cdot \sqrt{1 + \frac{1}{k^2} \partial_u^2 + \frac{1}{k^2} \partial_{y_1}^2} \right\} \phi$$

$$\Delta_5 = \partial_{x_1}^2 + \partial_{y_1}^2 + \partial_u^2 + \partial_v^2 + \partial_z^2$$

$$\cdots (2-2-6)$$

Expression (2-2-6) is summarized to obtain expression (2-2-7) below.
[Math. 18]

$$\left\{ \left( \Delta_5 + 2k^2 \right)^2 - 4 \left( k^2 + \partial_{x_1}^2 + \partial_v^2 \right) \left( k^2 + \partial_u^2 + \partial_{y_1}^2 \right) \right\} \phi = 0$$

$$\cdots (2-2-7)$$

Expression (2-2-7) is a scattering field equation that the scattering field function satisfies. Expression (2-2-7) is further summarized to obtain expression (2-2-8) below.
[Math. 19]

$$\left\{ \Delta_5^2 + 4k^2 \partial_z^2 - 4 \left( \partial_{x_1}^2 + \partial_v^2 \right) \left( \partial_u^2 + \partial_{y_1}^2 \right) \right\} \phi = 0$$

$$\cdots (2-2-8)$$

By Fourier transforming both sides of expression (2-2-8), expression (2-2-9) is obtained.
[Math. 20]

$$\int_{-\infty}^{\infty}\int_{-\infty}^{\infty}\int_{-\infty}^{\infty}\int_{-\infty}^{\infty} e^{ik_{x_1}x_1+ik_{y_1}y_1+ik_{x_2}x_2+ik_{y_2}y_2}$$

$$\cdot\left\{\Delta_5{}^2+4k^2\partial_z{}^2-4\left(\partial_{x_1}{}^2+\partial_v{}^2\right)\left(\partial_u{}^2+\partial_{y_1}{}^2\right)\right\}\phi\ dx_1dy_1dx_2dy_2=0$$

$$\cdots(2-2-9)$$

When the differential in the z direction is expressed as $D_z$, a one-variable differential equation expressed by expression (2-2-10) below is obtained from expression (2-2-9).
[Math. 21]

$$\left\{\left(D_z{}^2-k_{x_1}{}^2-k_{y_1}{}^2-k_u{}^2-k_v{}^2\right)^2+4k^2D_z{}^2-4\left(k_{x_1}{}^2+k_v{}^2\right)\left(k_u{}^2+k_{y_1}{}^2\right)\right\}$$

$$\cdot\tilde{\phi}(k_{x_1},k_{y_1},k_{x_2},k_{y_2},z,k)=0$$

$$\cdots(2-2-10)$$

When the solution form of expression (2-2-10) is assumed to be $e^{sz}$, expression (2-2-11) below is obtained for s.
[Math. 22]

$$\left(s^2-k_{x_1}{}^2-k_{y_1}{}^2-k_u{}^2-k_v{}^2\right)^2+4k^2s^2-4\left(k_{x_1}{}^2+k_v{}^2\right)\left(k_u{}^2+k_{y_1}{}^2\right)=0$$

$$\cdots(2-2-11)$$

Four solutions expressed by expression (2-2-12) below are obtained from expression (2-2-11).
[Math. 23]

$$s=\pm i\left\{\sqrt{k^2-\left\{k_{x_1}{}^2+(k_{y_2}+\beta k)^2\right\}}\pm\sqrt{k^2-\left\{k_{y_1}{}^2+(k_{x_2}+\alpha k)^2\right\}}\right\}$$

$$\cdots(2-2-12)$$

Therefore, the scattering field function, which is the solution of expression (2-2-8), is expressed as given by expression (2-2-13) below.
[Math. 24]

$$\phi(x_1,y_1,x_2,y_2,z,k)$$

$$=\frac{1}{(2\pi)^4}\int_{-\infty}^{\infty}\int_{-\infty}^{\infty}\int_{-\infty}^{\infty}\int_{-\infty}^{\infty}e^{-i(k_{x_1}x_1+k_{y_1}y_1)}e^{-i(k_{x_2}x_2+k_{y_2}y_2)}a(k_{x_1},k_{y_1},k_{x_2},k_{y_2},k)$$

$$\cdot e^{iz\left\{\sqrt{k^2-\left\{k_{x_1}{}^2+(k_{y_2}+\beta k)^2\right\}}\pm\sqrt{k^2-\left\{k_{y_1}{}^2+(k_{x_2}+\alpha k)^2\right\}}\right\}}dk_{x_1}dk_{y_1}dk_{x_2}dk_{y_2}$$

$$\cdots(2-2-13)$$

Transmitter array 111 transmits a wave in which a plurality of waves are integrated by a plurality of transmitters 101 connected in the $\gamma$-axis direction. Receiver array 112 receives a wave in which a plurality of waves are integrated by a plurality of receivers 102 connected in the x-axis direction. Therefore, by using the sum of expression (2-2-13) with respect

to variables $x_2$ and $y_2$, expression (2-2-14) below is derived.
[Math. 25]

$$\Phi(x_1, y_1, z, \alpha, \beta, k)$$

$$= \sum_{m=-\infty}^{\infty} \sum_{n=-\infty}^{\infty} \phi(x_1, y_1, m\Delta x, n\Delta y, z, k)$$

$$= \frac{1}{(2\pi)^4} \int_{-\infty}^{\infty}\int_{-\infty}^{\infty}\int_{-\infty}^{\infty}\int_{-\infty}^{\infty} e^{-i(k_{x_1}x_1+k_{y_1}y_1)} \left\{ \sum_{m=-\infty}^{\infty} \sum_{n=-\infty}^{\infty} e^{-i(k_{x_2}m\Delta x+k_{y_2}n\Delta y)} \right\} a(k_{x_1}, k_{y_1}, k_{x_2}, k_{y_2}, k)$$

$$\cdot e^{iz\left\{ \sqrt{k^2-\left\{k_{x_1}^2+(k_{y_2}+\beta k)^2\right\}} \pm \sqrt{k^2-\left\{k_{y_1}^2+(k_{x_2}+\alpha k)^2\right\}} \right\}} dk_{x_1} dk_{y_1} dk_{x_2} dk_{y_2}$$

$$\cdots (2-2-14)$$

In expression (2-2-14), $m\Delta x$ corresponds to $x_2$, and $n\Delta y$ corresponds to $y_2$. Here, each of m and n is an integer. Next, to remove $\Sigma$ from expression (2-2-14), a formula expressed by expression (2-2-15) below is used.
[Math. 26]

$$2\pi\delta(x) = \sum_{n=-\infty}^{\infty} e^{inx}$$

$$\cdots (2-2-15)$$

Expression (2-2-16) below is immediately obtained from expression (2-2-15).
[Math. 27]

$$\sum_{m=-\infty}^{\infty} e^{-ik_{x_2}m\Delta x} = 2\pi\delta(k_{x_2}\Delta x)$$

$$\sum_{n=-\infty}^{\infty} e^{-ik_{x_2}n\Delta y} = 2\pi\delta(k_{y_2}\Delta y)$$

$$\cdots (2-2-16)$$

When the phase factors of the phased array are $\alpha$ and $\beta$, the function $a(k_{x1}, k_{y1}, k_{x2}, k_{y2}, k)$ will be denoted as $a_{\alpha,\beta}(k_{x1}, k_{y1}, k_{x2}, k_{y2}, k)$ for clarity in the following. Moreover, $a_{\alpha,\beta}(k_{x1}, k_{y1}, k_{x2}, k_{y2}, k)$ at $k_{x2}=0$ and $k_{y2}=0$ is expressed as $b(k_{x1}, k_{y1}, \alpha, \beta, k) = a_{\alpha,\beta}(k_{x1}, k_{y1}, 0, 0, k)$.
From expression (2-2-14), expression (2-2-17) below is obtained using expression (2-2-16).
[Math. 28]

$$\Phi(x_1, y_1, z, \alpha, \beta, k)$$

$$= \frac{1}{(2\pi)^2} \int_{-\infty}^{\infty}\int_{-\infty}^{\infty}\int_{-\infty}^{\infty}\int_{-\infty}^{\infty} e^{-i(k_{x_1}x_1+k_{y_1}y_1)}\delta(k_{x_2}\Delta x)\delta(k_{y_2}\Delta y)a_{\alpha,\beta}(k_{x_1}, k_{y_1}, k_{x_2}, k_{y_2}, k)$$

$$\cdot e^{iz\left\{\sqrt{k^2-\left\{k_{x_1}^2+(k_{y_2}+\beta k)^2\right\}}\pm\sqrt{k^2-\left\{k_{y_1}^2+(k_{x_2}+\alpha k)^2\right\}}\right\}} dk_{x_1} dk_{y_1} dk_{x_2} dk_{y_2}$$

$$= \frac{1}{(2\pi)^2 \Delta x \Delta y} \int_{-\infty}^{\infty}\int_{-\infty}^{\infty} e^{-i(k_{x_1}x_1+k_{y_1}y_1)}b(k_{x_1}, k_{y_1}, \alpha, \beta, k)$$

$$\cdot e^{iz\left\{\sqrt{k^2-\left\{k_{x_1}^2+\beta^2 k^2\right\}}\pm\sqrt{k^2-\left\{k_{y_1}^2+\alpha^2 k^2\right\}}\right\}} dk_{x_1} dk_{y_1}$$

$$\cdots (2-2-1\,7)$$

Expression (2-2-17) corresponds to the scattering field function of the phased array.

The measurement data is obtained at z = 0. By substituting z = 0 in expression (2-2-17), Q indicating the measurement data is expressed by expression (2-2-18) below.

[Math. 29]

$$Q(x_1, y_1, \alpha, \beta, k) = \frac{1}{(2\pi)^2 \Delta x \Delta y} \int_{-\infty}^{\infty}\int_{-\infty}^{\infty} e^{-i(k_{x_1}x_1+k_{y_1}y_1)}b(k_{x_1}, k_{y_1}, \alpha, \beta, k)dk_{x_1} dk_{y_1}$$

$$\cdots (2-2-1\,8)$$

Expression (2-2-19) below is obtained by inverse Fourier transform of expression (2-2-18).

[Math. 30]

$$b(k_{x_1}, k_{y_1}, \alpha, \beta, k) = \Delta x \Delta y \tilde{Q}(k_{x_1}, k_{y_1}, \alpha, \beta, k)$$

$$\cdots (2-2-1\,9)$$

Accordingly, the scattering field function is obtained as given by expression (2-2-20) below.

[Math. 31]

$$\Phi(x_1, y_1, z, \alpha, \beta, k) = \frac{1}{(2\pi)^2} \int_{-\infty}^{\infty}\int_{-\infty}^{\infty} e^{-i(k_{x_1}x_1+k_{y_1}y_1)}\tilde{Q}(k_{x_1}, k_{y_1}, \alpha, \beta, k)$$

$$\cdot e^{iz\left\{\sqrt{k^2-\left\{k_{x_1}^2+\beta^2 k^2\right\}}\pm\sqrt{k^2-\left\{k_{y_1}^2+\alpha^2 k^2\right\}}\right\}} dk_{x_1} dk_{y_1}$$

$$\cdots (2-2-2\,0)$$

The scattering field function of expression (2-2-20) corresponds to the imaging function. When expression (2-2-20) is used as the imaging function, an image is obtained for each phase factor ($\alpha$, $\beta$) of the phased array. However, in principle, a single image should be obtained as an image indicating the object, regardless of the phase factor ($\alpha$, $\beta$). Therefore, expression (2-2-20) is synthesized with respect to ($\alpha$, $\beta$). As a result, the imaging function $\rho(x, y, z)$ is obtained as given by expression (2-2-21) below.

[Math. 32]

$$\rho(x,y,z)$$

$$= \frac{1}{(2\pi)^2} \int\limits_0^\infty \int\limits_{-\infty}^\infty \int\limits_{-\infty}^\infty e^{-i(k_{x_1}x + k_{y_1}y)} \sum_\alpha \sum_\beta \left\{ \tilde{Q}(k_{x_1}, k_{y_1}, \alpha, \beta, k) \cdot e^{ik_z z} \right\} dk\, dk_{x_1} dk_{y_1}$$

$$k_z = \sqrt{k^2 - \left\{ k_{x_1}^2 + \beta^2 k^2 \right\}} \pm \sqrt{k^2 - \left\{ k_{y_1}^2 + \alpha^2 k^2 \right\}}$$

$$\cdots (2-2-21)$$

Moreover, by converting the integration variable from dk to $dk_z$, expression (2-2-22) below is obtained.
[Math. 33]

$$\rho(x,y,z)$$

$$= \frac{1}{(2\pi)^2} \int\limits_0^\infty \int\limits_{-\infty}^\infty \int\limits_{-\infty}^\infty e^{-i(k_{x_1}x + k_{y_1}y)} \sum_\alpha \sum_\beta \left\{ \tilde{Q}(k_{x_1}, k_{y_1}, \alpha, \beta, k) \cdot e^{ik_z z} \right\} \left( \frac{dk}{dk_z} \right) dk_{x_1} dk_{y_1} dk_z$$

$$k_z = \sqrt{k^2 - \left\{ k_{x_1}^2 + \beta^2 k^2 \right\}} \pm \sqrt{k^2 - \left\{ k_{y_1}^2 + \alpha^2 k^2 \right\}}$$

$$\cdots (2-2-22)$$

Here, the relationship between k and $k_z$ is expressed as given by expression (2-2-23) below.
[Math. 34]

$$k = \frac{1}{\sqrt{1-\alpha^2}} \sqrt{ k_{y_1}^2 + \left\{ \frac{-\frac{1-\beta^2}{1-\alpha^2} k_{y_1}^2 + k_{x_1}^2 + k_z^2}{k_z + \sqrt{k_z^2 - \left( \frac{1-\beta^2}{1-\alpha^2} - 1 \right) \left( \frac{1-\beta^2}{1-\alpha^2} k_{y_1}^2 - k_{x_1}^2 - k_z^2 \right)}} \right\}^2 }$$

$$\cdots (2-2-23)$$

Also, expression (2-2-24) below is used for the conversion of the integration variable in expression (2-2-22).
[Math. 35]

$$\frac{dk}{dk_z} = \frac{1}{\frac{(1-\beta^2)k}{\sqrt{(1-\beta^2)k^2 - k_{x_1}^2}} + \frac{(1-\alpha^2)k}{\sqrt{(1-\alpha^2)k^2 - k_{y_1}^2}}}$$

$$\cdots (2-2-24)$$

The imaging device can image an object in the measurement area from the measurement data obtained by two-dimensional transceiver element array 150 arranged on a flat surface, by using the imaging function of expression (2-2-21) or expression (2-2-22). Here, two-dimensional transceiver element array 150 includes one or more transmitter arrays 111 and one or more receiver arrays 112.

<2-3. Relationship Between k and $k_z$>

The relationship between k and $k_z$ shown in expression (2-2-23) is derived as follows.

First, expression (2-3-1) below is derived from expression (2-2-21).

[Math. 36]

$$k_z = \sqrt{k^2 - \left\{k_{x_1}^2 + \beta^2 k^2\right\}} \pm \sqrt{k^2 - \left\{k_{y_1}^2 + \alpha^2 k^2\right\}}$$

$$= \sqrt{(1-\beta^2)k^2 - k_{x_1}^2} + \sqrt{(1-\alpha^2)k^2 - k_{y_1}^2}$$

$$\cdots (2-3-1)$$

To simplify the notation, variables are temporarily substituted as given by expression (2-3-2) below.

[Math. 37]

$$1 - \beta^2 \Rightarrow \beta, \quad 1 - \alpha^2 \Rightarrow \alpha, \quad k_{x_1}^2 \Rightarrow a, \quad k_{y_1}^2 \Rightarrow b, \quad k^2 \Rightarrow k$$

$$\cdots (2-3-2)$$

Expression (2-3-1) is expressed, using expression (2-3-2), as given by expression (2-3-3) below.

[Math. 38]

$$\sqrt{\beta k - a} + \sqrt{\alpha k - b} = k_z$$

$$\cdots (2-3-3)$$

Furthermore, variables as given by expression (2-3-4) below are introduced for expression (2-3-3).

[Math. 39]

$$x = \sqrt{k'} = \sqrt{\alpha k - b}$$

$$\cdots (2-3-4)$$

As a result, the equation represented by expression (2-3-5) below is obtained.

[Math. 40]

$$\left(\frac{\beta}{\alpha} - 1\right)x^2 + 2k_z x + \frac{\beta}{\alpha}b - a - k_z^2 = 0$$

$$\cdots (2-3-5)$$

Expression (2-3-6) is obtained from expression (2-3-5).

[Math. 41]

$$x = \frac{-k_z \pm \sqrt{k_z{}^2 - \left(\frac{\beta}{\alpha} - 1\right)\left(\frac{\beta}{\alpha}b - a - k_z{}^2\right)}}{\frac{\beta}{\alpha} - 1}$$

$$= \frac{-\frac{\beta}{\alpha}b + a + k_z{}^2}{k_z + \sqrt{k_z{}^2 - \left(\frac{\beta}{\alpha} - 1\right)\left(\frac{\beta}{\alpha}b - a - k_z{}^2\right)}}$$

$$\cdots (2-3-6)$$

In expression (2-3-6), the variables that were replaced by expression (2-3-2) are restored to their original form by expression (2-3-7) below.
[Math. 42]

$$1 - \beta^2 \Leftarrow \beta, \quad 1 - \alpha^2 \Leftarrow \alpha, \quad k_{x_1}{}^2 \Leftarrow a, \quad k_{y_1}{}^2 \Leftarrow b, \quad k^2 \Leftarrow k$$

$$\cdots (2-3-7)$$

Ultimately, expression (2-3-8) below is obtained.
[Math. 43]

$$k = \frac{1}{\sqrt{1-\alpha^2}} \sqrt{k_{y_1}{}^2 + \left\{ \frac{-\frac{1-\beta^2}{1-\alpha^2}k_{y_1}{}^2 + k_{x_1}{}^2 + k_z{}^2}{k_z + \sqrt{k_z{}^2 - \left(\frac{1-\beta^2}{1-\alpha^2} - 1\right)\left(\frac{1-\beta^2}{1-\alpha^2}k_{y_1}{}^2 - k_{x_1}{}^2 - k_z{}^2\right)}} \right\}^2}$$

$$\cdots (2-3-8)$$

Moreover, as in expression (2-3-1), $k_z$ is expressed as given by expression (2-3-9) below.
[Math. 44]

$$k_z = \sqrt{(1-\beta^2)k^2 - k_{x_1}{}^2} + \sqrt{(1-\alpha^2)k^2 - k_{y_1}{}^2}$$

$$\cdots (2-3-9)$$

Expression (2-3-10) below is obtained by differentiation of both sides of expression (2-3-9).
[Math. 45]

$$dk_z = \left\{ \frac{(1-\beta^2)k}{\sqrt{(1-\beta^2)k^2 - k_{x_1}^2}} + \frac{(1-\alpha^2)k}{\sqrt{(1-\alpha^2)k^2 - k_{y_1}^2}} \right\} dk$$

$$\cdots (2-3-10)$$

Expression (2-2-24) above is derived from expression (2-3-10).

<3. Imaging Device Using Two-dimensional Transceiver Element Array on Curved Surface>

In this chapter, the imaging device uses a two-dimensional transceiver element array 150 on a curved surface. For example, a polymer piezoelectric element of, for example, polyvinylidene fluoride is configured as a thin film having a film thickness of 40 to 100 $\mu$m, inclusive. Charges proportional to the stretching of the thin film appear on both surfaces of the thin film. When such a thin film is used as a piezoelectric element in two-dimensional transceiver element array 150, depending on the stretching of the thin film, two-dimensional transceiver element array 150 may be arranged on a curved surface.

FIG. 9 is a conceptual diagram illustrating two-dimensional transceiver element array 150 arranged on a curved surface. In this chapter, a reconstructed image of the object is generated from measurement data when two-dimensional transceiver element array 150 is arranged on a curved surface as illustrated in FIG. 9.

FIG. 10 is a schematic diagram illustrating the positions of transmitter array 111 and receiver array 112 in two-dimensional transceiver element array 150 arranged on a curved surface. For example, two-dimensional transceiver element array 150 is arranged on a curved surface expressed by z = f(x, y). Two-dimensional transceiver element array 150 operates as an N×N orthogonal curved surface array. In this example, a scattering field function and an imaging function as shown below are derived.

First, a scattering field function expressed as given by expression (3-1-1) below is examined.
[Math. 46]

$$\phi(x_1, y_1, x_2, y_2, z_1, z_2, \omega) = \iint_D \frac{e^{ik\rho_1}}{\rho_1} \frac{e^{ik\rho_2}}{\rho_2} \varepsilon(\xi, \eta, \zeta) d\xi d\eta d\zeta$$

$$\cdots (3-1-1)$$

Here, $(x_1, y_2, z_1)$ corresponds to transmission point $P_1$, and $(x_2, y_1, z_2)$ corresponds to reception point $P_2$. $\omega$ represents the angular frequency of the wave. k represents the wavenumber of the wave. $\varepsilon(\xi, \eta, \zeta)$ corresponds to the reflectance at point $P(\xi, \eta, \zeta)$ of the object. Point $P(\xi, \eta, \zeta)$ corresponds to the reflection point. Note that $\varepsilon(\xi, \eta, \zeta)$ is unknown. It is assumed here that the time factor is proportional to exp(-i$\omega$t). Moreover, $\rho_1$ and $\rho_2$ are expressed as given by expression (3-1-2) below.
[Math. 47]

$$\rho_1 = \sqrt{(x_1 - \xi)^2 + (y_2 - \eta)^2 + (z_1 - \varsigma)^2}$$

$$\rho_2 = \sqrt{(x_2 - \xi)^2 + (y_1 - \eta)^2 + (z_2 - \varsigma)^2}$$

$$\cdots (3-1-2)$$

The equation satisfied by $\varphi(x_1, y_1, x_2, y_2, z_1, z_2, \omega)$ is derived as follows.

First, the kernel function in the integrand term of expression (3-1-1) is represented as $\varphi$ in expression (3-1-3) below.
[Math. 48]

$$\phi = \frac{e^{ik\rho_1}}{\rho_1}\frac{e^{ik\rho_2}}{\rho_2}$$

$$\cdots (3-1-3)$$

Next, a partial differential equation that has expression (3-1-3) as an asymptotic solution is examined. Thus, calculation is performed while ignoring a high-order term with respect to 1/p obtained as a result of differentiation. Here, an abridged notation for differentiation is defined by expression (3-1-4).
[Math. 49]

$$\frac{\partial}{\partial t} \to \partial_t, \frac{\partial}{\partial x_1} \to \partial_{x_1}, \frac{\partial}{\partial x_2} \to \partial_{x_2}, \frac{\partial}{\partial y_1} \to \partial_{y_1}, \frac{\partial}{\partial y_2} \to \partial_{y_2}, \frac{\partial}{\partial z_1} \to \partial_{z_1}, \frac{\partial}{\partial z_2} \to \partial_{z_2}$$

$$\cdots (3-1-4)$$

The result of differentiation of each order of $\varphi$ is represented by expression (3-1-5) below.
[Math. 50]

$$\partial_{x_1}\phi = ik\frac{(x_1-\xi)}{\rho_1}\phi + o(\rho^{-3}) \qquad \partial_{y_1}\phi = ik\frac{y_1-\eta}{\rho_2}\phi + o(\rho^{-3})$$

$$\partial_{x_2}\phi = ik\left(\frac{x_2-\xi}{\rho_2}\right)\phi + o(\rho^{-3}) \qquad \partial_{y_2}\phi = ik\left(\frac{y_2-\eta}{\rho_1}\right)\phi + o(\rho^{-3})$$

$$\partial_{z_1}\phi = ik\left(\frac{z_1-\zeta}{\rho_1}\right)\phi + o(\rho^{-3}) \qquad \partial_{z_2}\phi = ik\left(\frac{z_2-\zeta}{\rho_2}\right)\phi + o(\rho^{-3})$$

$$\partial_{x_1}\partial_{x_1}\phi = (ik)^2\frac{(x_1-\xi)^2}{\rho_1^{\,2}}\phi + o(\rho^{-3})$$

$$\partial_{x_2}\partial_{x_2}\phi = (ik)^2\left(\frac{x_2-\xi}{\rho_2}\right)^2\phi + o(\rho^{-3})$$

$$\partial_{y_1}\partial_{y_1}\phi = (ik)^2\frac{(y_1-\eta)^2}{\rho_2^{\,2}}\phi + o(\rho^{-3})$$

$$\partial_{y_2}\partial_{y_2}\phi = (ik)^2\left(\frac{y_2-\eta}{\rho_1}\right)^2\phi + o(\rho^{-3})$$

$$\partial_{z_1}\partial_{z_1}\phi = (ik)^2\left(\frac{z_1-\zeta}{\rho_1}\right)^2\phi + o(\rho^{-3})$$

$$\partial_{z_2}\partial_{z_2}\phi = (ik)^2\left(\frac{z_2-\zeta}{\rho_2}\right)^2\phi + o(\rho^{-3})$$

$$\cdots (3-1-5)$$

Hereinafter, the complexity O(*) is omitted. In accordance with the sum of six differential equations of the second order, expression (3-1-6) below is obtained.
[Math. 51]

$$\Delta_6\phi = (\partial_{x_1}^{\,2} + \partial_{x_2}^{\,2} + \partial_{y_1}^{\,2} + \partial_{y_2}^{\,2} + \partial_{z_1}^{\,2} + \partial_{z_2}^{\,2})\phi$$
$$= 2(ik)^2\phi$$

$$\cdots (3-1-6)$$

By Fourier transforming both sides of expression (3-1-6), expression (3-1-7) below is obtained.
[Math. 52]

$$\int_{-\infty}^{\infty}\int_{-\infty}^{\infty}\int_{-\infty}^{\infty}\int_{-\infty}^{\infty} e^{ik_{x_1}x_1+ik_{y_1}y_1+ik_{x_2}x_2+ik_{y_2}y_2}\left\{\Delta_6 - 2(ik)^2\right\}\phi \, dx_1 dy_1 dx_2 dy_2 = 0$$

$$\cdots (3-1-7)$$

By expressing partial differentials with respect to $z_1$; and $z_2$ as $D_{z1}$ and $D_{z2}$, respectively, expression (3-1-8) below is obtained.
[Math. 53]

$$\left\{(D_{z_1}{}^2 + D_{z_2}{}^2 - k_{x_1}{}^2 - k_{y_1}{}^2 - k_{x_2}{}^2 - k_{y_2}{}^2) + 2k^2\right\}\tilde{\phi} = 0$$

$$\cdots (3-1-8)$$

Next, solving the equation given by expression (3-1-8) is examined. This equation includes two variables, $z_1$ and $z_2$. Therefore, it is difficult to determine the solution solely from the boundary conditions. However, the solution for the case where z1 = z2 has already been determined. The solution to expression (3-1-8) has consistency with the solution for the case where z1 = z2. Therefore, the solution to expression (3-1-8) is uniquely determined by two conditions: the boundary conditions and the consistency condition. For example, a solution to expression (3-1-8) is assumed as given by expression (3-1-9) below.
[Math. 54]

$$E(k_{x_1}, k_{x_2}, k_{y_1}, k_{y_2}, z_1, z_2) = \exp(is_1 z_1)\exp(is_2 z_2)$$

$$\cdots (3-1-9)$$

In the case where z1 = z2, by substituting expression (3-1-9) in expression (3-1-8), expression (3-1-10) below is obtained.
[Math. 55]

$$s_1{}^2 + s_2{}^2 + k_{x_1}{}^2 + k_{y_1}{}^2 + k_{x_2}{}^2 + k_{y_2}{}^2 - 2k^2 = 0$$

$$\cdots (3-1-1\,0)$$

Based on expressions (2-2-12) and (2-2-13) in the case where each of phase factors $\alpha$ and $\beta$ of the phased array is 0, and on the use of backscattering, expression (3-1-11) below is obtained.
[Math. 56]

$$s_1 + s_2 = \sqrt{k^2 - k_{x_1}{}^2 - k_{y_2}} + \sqrt{k^2 - k_{y_1}{}^2 - k_{x_2}}$$

$$\cdots (3-1-1\,1)$$

From expressions (3-1-10) and (3-1-11), $s_1$ and $s_2$ are obtained as given by expression (3-1-12) below.
[Math. 57]

$$s_1 = \sqrt{k^2 - k_{x_1}{}^2 - k_{y_2}}$$

$$s_2 = \sqrt{k^2 - k_{y_1}{}^2 - k_{x_2}}$$

$$\cdots (3-1-1\,2)$$

Therefore, the solution of equation (3-1-6) is expressed as given by expression (3-1-13) below.
[Math. 58]

$$\phi(x_1, y_1, x_2, y_2, z_1, z_2, k)$$

$$= \frac{1}{(2\pi)^4} \int_{-\infty}^{\infty} \int_{-\infty}^{\infty} \int_{-\infty}^{\infty} \int_{-\infty}^{\infty} e^{-i(k_{x_1}x_1 + k_{y_1}y_1)} e^{-i(k_{x_2}x_2 + k_{y_2}y_2)} a(k_{x_1}, k_{y_1}, k_{x_2}, k_{y_2}, k)$$

$$\cdot e^{iz_1\sqrt{k^2 - k_{x_1}^2 - k_{y_2}^2}} e^{iz_2\sqrt{k^2 - k_{y_1}^2 - k_{x_2}^2}} dk_{x_1} dk_{y_1} dk_{x_2} dk_{y_2}$$

$$\cdots (3-1-13)$$

Next, deriving $a(k_{x1}, k_{y1}, k_{x2}, k_{y2}, k)$ in expression (3-1-13) is examined. Therefore, variables in expression (3-1-14) below are used.
[Math. 59]

$$z_1 = z + v_2(x_1, y_2)$$
$$z_2 = z + u_2(y_1, x_2)$$
$$\cdots (3-1-14)$$

In expression (3-1-14), $u_2$ and $v_2$ are variables representing the shape of the curved surface, and $z$ represents the position of the reference plane. $u_2$ is a variable that depends on $(x_1, y_2)$ and corresponds to a function that uses $(x_1, y_2)$ as input. $v_2$ is a variable that depends on $(y_1, x_2)$ and corresponds to a function that uses $(y_1, x_2)$ as input. By substituting expression (3-1-14) in expression (3-1-13), expression (3-1-15) below is obtained.
[Math. 60]

$$\phi(x_1, y_1, x_2, y_2, z_1, z_2, k)$$

$$= \frac{1}{(2\pi)^4} \int_{-\infty}^{\infty} \int_{-\infty}^{\infty} \int_{-\infty}^{\infty} \int_{-\infty}^{\infty} e^{-i(k_{x_1}x_1 + k_{y_1}y_1)} e^{-i(k_{x_2}x_2 + k_{y_2}y_2)} a(k_{x_1}, k_{y_1}, k_{x_2}, k_{y_2}, k)$$

$$\cdot e^{i(z+v_2)\sqrt{k^2 - k_{x_1}^2 - k_{y_2}^2}} e^{i(z+u_2)\sqrt{k^2 - k_{y_1}^2 - k_{x_2}^2}} dk_{x_1} dk_{y_1} dk_{x_2} dk_{y_2}$$

$$\cdots (3-1-15)$$

Transmitter array 111 transmits a wave in which a plurality of waves are integrated by a plurality of transmitters 101 connected in the $\gamma$-axis direction. Receiver array 112 receives a wave in which a plurality of waves are integrated by a plurality of receivers 102 connected in the x-axis direction. Therefore, by using the sum of expression (3-1-15) with respect to variables $x_2$ and $y_2$, expression (3-1-16) below is derived.
[Math. 61]

$$\Phi(x_1, y_1, z, k)$$

$$= \sum_{x_2, y_2} \phi(x_1, y_1, x_2, y_2, z_1, z_2, k)$$

$$= \frac{1}{(2\pi)^4} \int_{-\infty}^{\infty} \int_{-\infty}^{\infty} \int_{-\infty}^{\infty} \int_{-\infty}^{\infty} e^{-i(k_{x_1}x_1 + k_{y_1}y_1)} \left\{ \sum_{x_2, y_2} e^{-i(k_{x_2}x_2 + k_{y_2}y_2)} e^{iv_2\sqrt{k^2 - k_{x_1}^2 - k_{y_2}^2}} e^{iu_2\sqrt{k^2 - k_{y_1}^2 - k_{x_2}^2}} \right\}$$

$$\cdot a(k_{x_1}, k_{y_1}, k_{x_2}, k_{y_2}, k) \, e^{iz\left(\sqrt{k^2 - k_{x_1}^2 - k_{y_2}^2} + \sqrt{k^2 - k_{y_1}^2 - k_{x_2}^2}\right)} \, dk_{x_1} dk_{y_1} dk_{x_2} dk_{y_2}$$

$$\cdots (3-1-16)$$

For example, measurement data corresponding to a reference plane of z=0 is obtained. This measurement data is examined as a boundary condition for deriving the unknown function $a(k_{x1}, k_{y1}, k_{x2}, k_{y2}, k)$. Here, based on expression (3-1-16), an integral equation as given by expression (3-1-17) below holds true. The left side corresponds to the measurement data and is known.
[Math. 62]

$$\Phi(x_1, y_1, 0, k)$$

$$= \frac{1}{(2\pi)^4} \int_{-\infty}^{\infty} \int_{-\infty}^{\infty} \int_{-\infty}^{\infty} \int_{-\infty}^{\infty} e^{-i(k_{x_1}x_1 + k_{y_1}y_1)} \left\{ \sum_{x_2, y_2} e^{-i(k_{x_2}x_2 + k_{y_2}y_2)} e^{iv_2\sqrt{k^2 - k_{x_1}^2 - k_{y_2}^2}} e^{iu_2\sqrt{k^2 - k_{y_1}^2 - k_{x_2}^2}} \right\}$$

$$\cdot a(k_{x_1}, k_{y_1}, k_{x_2}, k_{y_2}, k) \, dk_{x_1} dk_{y_1} dk_{x_2} dk_{y_2}$$

$$\cdots (3-1-17)$$

For example, when the position of the x-coordinate of transmitter array 111 is expressed as $x_I$, and the position of the y-coordinate of receiver array 112 is expressed as $y_J$, measurement data cannot be obtained where $x_1 \neq x_I$ or $y_1 \neq y_J$. Accordingly, expression (3-1-17) can be interpreted as given by expression (3-1-18) below.
[Math. 63]

$$\Phi(x_1, y_1, 0, k)\delta(x_1 - x_I)\delta(y_1 - y_J)$$

$$= \frac{1}{(2\pi)^4} \int_{-\infty}^{\infty} \int_{-\infty}^{\infty} \int_{-\infty}^{\infty} \int_{-\infty}^{\infty} e^{-i(k_{x_1}x_1 + k_{y_1}y_1)}$$

$$\cdot \left\{ \sum_{x_2, y_2} e^{-i(k_{x_2}x_2 + k_{y_2}y_2)} e^{iv_2(x_I, y_2)\sqrt{k^2 - k_{x_1}^2 - k_{y_2}^2}} e^{iu_2(y_J, x_2)\sqrt{k^2 - k_{y_1}^2 - k_{x_2}^2}} \right\}$$

$$\cdot a_{I,J}(k_{x_1}, k_{y_1}, k_{x_2}, k_{y_2}, k) \, dk_{x_1} dk_{y_1} dk_{x_2} dk_{y_2}$$

$$\cdots (3-1-18)$$

Expression (3-1-19) below is obtained by performing inverse Fourier transform of both sides of expression (3-1-18) with respect to $x_1$, $y_1$.
[Math. 64]

$$\int_{-\infty}^{\infty} e^{i(k_{x_1}'x_1 + k_{y_1}'y_1)} \Phi(x_1, y_1, 0, k) \delta(x_1 - x_I) \delta(y_1 - y_J) dx_1 dy_1$$

$$= \frac{1}{(2\pi)^4} \int_{-\infty}^{\infty} \int_{-\infty}^{\infty} \int_{-\infty}^{\infty} \int_{-\infty}^{\infty} \int_{-\infty}^{\infty} \int_{-\infty}^{\infty} e^{i(k_{x_1}'x_1 + k_{y_1}'y_1)} e^{-i(k_{x_1}x_1 + k_{y_1}y_1)}$$

$$\cdot \left\{ \sum_{x_2, y_2} e^{-i(k_{x_2}x_2 + k_{y_2}y_2)} e^{iv_2(x_I, y_2)\sqrt{k^2 - k_{x_1}^2 - k_{y_2}^2}} e^{iu_2(y_J, x_2)\sqrt{k^2 - k_{y_1}^2 - k_{x_2}^2}} \right\}$$

$$\cdot a_{I,J}(k_{x_1}, k_{y_1}, k_{x_2}, k_{y_2}, k) \, dk_{x_1} dk_{y_1} dk_{x_2} dk_{y_2} dx_1 dy_1$$

$$\cdots (3-1-19)$$

Expression (3-1-20) is obtained from expression (3-1-19).
[Math. 65]

$$e^{i(k_{x_1}'x_I + k_{y_1}'y_J)} \Phi(x_I, y_J, 0, k)$$

$$= \frac{1}{(2\pi)^2} \int_{-\infty}^{\infty} \int_{-\infty}^{\infty} \int_{-\infty}^{\infty} \int_{-\infty}^{\infty} \delta(k_{x_1} - k_{x_1}') \delta(k_{y_1} - k_{y_1}')$$

$$\cdot \left\{ \sum_{x_2, y_2} e^{-i(k_{x_2}x_2 + k_{y_2}y_2)} e^{iv_2(x_I, y_2)\sqrt{k^2 - k_{x_1}^2 - k_{y_2}^2}} e^{iu_2(y_J, x_2)\sqrt{k^2 - k_{y_1}^2 - k_{x_2}^2}} \right\}$$

$$\cdot a_{I,J}(k_{x_1}, k_{y_1}, k_{x_2}, k_{y_2}, k) \, dk_{x_1} dk_{y_1} dk_{x_2} dk_{y_2}$$

$$= \frac{1}{(2\pi)^2} \int_{-\infty}^{\infty} \int_{-\infty}^{\infty} \left\{ \sum_{x_2, y_2} e^{-i(k_{x_2}x_2 + k_{y_2}y_2)} e^{iv_2(x_I, y_2)\sqrt{k^2 - k_{x_1}'^2 - k_{y_2}^2}} e^{iu_2(y_J, x_2)\sqrt{k^2 - k_{y_1}'^2 - k_{x_2}^2}} \right\}$$

$$\cdot a_{I,J}(k_{x_1}', k_{y_1}', k_{x_2}, k_{y_2}, k) \, dk_{x_2} dk_{y_2}$$

$$\cdots (3-1-20)$$

Furthermore, expression (3-1-21) is obtained from expression (3-1-20).
[Math. 66]

$$\frac{1}{(2\pi)^2} \int_{-\infty}^{\infty} \int_{-\infty}^{\infty} \left\{ \sum_{x_2, y_2} e^{-i(k_{x_2}x_2 + k_{y_2}y_2)} e^{iv_2(x_I, y_2)\sqrt{k^2 - k_{x_1}^2 - k_{y_2}^2}} e^{iu_2(y_J, x_2)\sqrt{k^2 - k_{y_1}^2 - k_{x_2}^2}} \right\}$$

$$\cdot a_{I,J}(k_{x_1}, k_{y_1}, k_{x_2}, k_{y_2}, k) \, dk_{x_2} dk_{y_2}$$

$$= e^{i(k_{x_1}x_I + k_{y_1}y_J)} \Phi(x_I, y_J, 0, k)$$

$$\cdots (3-1-21)$$

Furthermore, expression (3-1-22) is obtained from expression (3-1-21).

[Math. 67]

$$\frac{1}{(2\pi)^2}\int_{-\infty}^{\infty}\int_{-\infty}^{\infty}\left\{\sum_{m=-\infty}^{\infty}\sum_{n=-\infty}^{\infty}e^{-i(k_{x_2}m\Delta x+k_{y_2}n\Delta y)}e^{iv_2(x_I,n\Delta y)\sqrt{k^2-k_{x_1}^2-k_{y_2}^2}}e^{iu_2(y_J,m\Delta x)\sqrt{k^2-k_{y_1}^2-k_{x_2}^2}}\right\}$$

$$\cdot a_{I,J}(k_{x_1},k_{y_1},k_{x_2},k_{y_2},k)\,dk_{x_2}\,dk_{y_2}$$

$$= e^{i(k_{x_1}x_I+k_{y_1}y_J)}\Phi(x_I,y_J,0,k)$$

$$\cdots(3-1-22)$$

The factor included in the integrand of the left side of expression (3-1-22) is expressed by $G_{I,J}(k_{x1}, k_{y1}, k_{x2}, k_{y2}, k)$ in expression (3-1-23) below. Furthermore, when the curvature of the curved surface is small, $G_{I,J}(k_{x1}, k_{y1}, k_{x2}, k_{y2}, k)$ is simplified as illustrated in the last line of expression (3-1-23) below.
[Math. 68]

$$G_{I,J}(k_{x_1},k_{y_1},k_{x_2},k_{y_2},k)$$

$$= \sum_{m=-\infty}^{\infty}\sum_{n=-\infty}^{\infty}e^{-i(k_{x_2}m\Delta x+k_{y_2}n\Delta y)}e^{iv_2(x_I,n\Delta y)\sqrt{k^2-k_{x_1}^2-k_{y_2}^2}}e^{iu_2(y_J,m\Delta x)\sqrt{k^2-k_{y_1}^2-k_{x_2}^2}}$$

$$\xrightarrow[u_2,v_2\to 0]{}\sum_{m=-\infty}^{\infty}\sum_{n=-\infty}^{\infty}e^{-i(k_{x_2}m\Delta x+k_{y_2}n\Delta y)}=(2\pi)^2\delta(k_{x_2}\Delta x)\delta(k_{y_2}\Delta y)$$

$$\cdots(3-1-23)$$

According to expression (3-1-23), it is possible to take the main terms in expression (3-1-22) outside the integral. Also, expression (3-1-24) below is derived from expression (3-1-22).
[Math. 69]

$$a_{I,J}(k_{x_1},k_{y_1},0,0,k)$$

$$= e^{i(k_{x_1}x_I+k_{y_1}y_J)}\Phi(x_I,y_J,0,k)$$

$$-\frac{1}{(2\pi)^2}\int_{-\infty}^{\infty}\int_{-\infty}^{\infty}\left[\sum_{m=-\infty}^{\infty}\sum_{n=-\infty}^{\infty}e^{-i(k_{x_2}m\Delta x+k_{y_2}n\Delta y)}\left\{e^{iv_2(x_I,n\Delta y)\sqrt{k^2-k_{x_1}^2-k_{y_2}^2}}\right.\right.$$

$$\left.\left.\cdot e^{iu_2(y_J,m\Delta x)\sqrt{k^2-k_{y_1}^2-k_{x_2}^2}}-1\right\}\right]a_{I,J}(k_{x_1},k_{y_1},k_{x_2},k_{y_2},k)\,dk_{x_2}\,dk_{y_2}$$

$$\cdots(3-1-24)$$

Expression (3-1-24) is a Fredholm integral equation of the second kind for $a(k_{x1}, k_{y1}, k_{x2}, k_{y2}, k)$. The equation of expression (3-1-24) indicates that the following substitution is possible.
[Math. 70]

$$a_{I,J}(k_{x_1},k_{y_1},k_{x_2},k_{y_2},k)=b_{I,J}(k_{x_1},k_{y_1},k)$$

$$\cdots(3-1-25)$$

Expression (3-1-26) below is obtained by taking $b_{I,J}(k_{x1}, k_{y1}, k)$ outside the integral in expression (3-1-22).
[Math. 71]

$$\frac{1}{(2\pi)^2} b_{I,J}(k_{x_1}, k_{y_1}, k) \int\limits_{-\infty}^{\infty}\int\limits_{-\infty}^{\infty} G_{I,J}(k_{x_1}, k_{y_1}, k_{x_2}, k_{y_2}, k)\, dk_{x_2} dk_{y_2}$$

$$= e^{i(k_{x_1}x_I + k_{y_1}y_J)}\Phi(x_I, y_J, 0, k)$$

$$\cdots (3-1-26)$$

$b_{I,J}(k_{x1}, k_{y1}, k)$ is immediately obtained from expression (3-1-26) as given by expression (3-1-27) below.
[Math. 72]

$$b_{I,J}(k_{x_1}, k_{y_1}, k) = \frac{e^{i(k_{x_1}x_I + k_{y_1}y_J)}\Phi(x_I, y_J, 0, k)}{\dfrac{1}{(2\pi)^2}\int\limits_{-\infty}^{\infty}\int\limits_{-\infty}^{\infty} G_{I,J}(k_{x_1}, k_{y_1}, k_{x_2}, k_{y_2}, k)\, dk_{x_2} dk_{y_2}}$$

$$\cdots (3-1-27)$$

By using the sum of $b_{I,J}(k_{x1}, k_{y1}, k)$ with respect to I and J, $b(k_{x1}, k_{y1}, k)$ is defined as given by expression (3-1-28) below.
[Math. 73]

$$b(k_{x_1}, k_{y_1}, k) = \sum_{I,J} b_{I,J}(k_{x_1}, k_{y_1}, k)$$

$$\cdots (3-1-28)$$

Using $b(k_{x1}, k_{y1}, k)$ defined by expression (3-1-28), the imaging function from expression (3-1-16) is derived as given by expression (3-1-29) below.
[Math. 74]

$$\rho(x_1, y_1, z) = \int\limits_{0}^{\infty} \Phi(x_1, y_1, z, k)\, dk$$

$$\Phi(x_1, y_1, z, k)$$

$$= \frac{1}{(2\pi)^4}\int\limits_{-\infty}^{\infty}\int\limits_{-\infty}^{\infty}\int\limits_{-\infty}^{\infty}\int\limits_{-\infty}^{\infty} e^{-i(k_{x_1}x_1 + k_{y_1}y_1)}\left\{\sum_{x_2, y_2} e^{-i(k_{x_2}x_2 + k_{y_2}y_2)} e^{iv_2\sqrt{k^2 - k_{x_1}^2 - k_{y_2}^2}} e^{iu_2\sqrt{k^2 - k_{y_1}^2 - k_{x_2}^2}}\right\}$$

$$\cdot b(k_{x_1}, k_{y_1}, k) e^{iz\left(\sqrt{k^2 - k_{x_1}^2 - k_{y_2}^2} + \sqrt{k^2 - k_{y_1}^2 - k_{x_2}^2}\right)}\, dk_{x_1} dk_{y_1} dk_{x_2} dk_{y_2}$$

$$\cdots (3-1-29)$$

The imaging device can image an object in the measurement area from the measurement data obtained by two-dimensional transceiver element array 150 arranged on a curved surface, by using the imaging function of expression (3-1-29).

<4. Variations>

In Chapters 2 and 3, transmitter array 111 and receiver array 112 that are orthogonal to each other are presented. Transmitter array 111 and receiver array 112 may be arranged along mutually different directions, rather than being orthogonal. Stated differently, a plurality of transmitters 101 may be connected in a first direction, and receivers 102 may be connected in a second direction different from the first direction. The second direction different from the first direction may also be expressed as a second direction not parallel to the first direction.

In Chapter 3, each of phase factors $\alpha$ and $\beta$ is 0. However, in Chapter 3, each of phase factors $\alpha$ and $\beta$ is not limited to being 0. In Chapter 2, each of phase factors $\alpha$ and $\beta$ may be 0. The description in Chapter 2 may be applied to Chapter 3, and the description in Chapter 3 may be applied to Chapter 2.

Even in the above variation, the scattering field function and the imaging function can be derived based on a theory of the same kind as the theory described above.

<5. Basic Configuration and Basic Operations of Imaging Device>

Based on the contents described above, the basic configuration and basic operations of an imaging device for imaging an object in a measurement area using measurement data of scattered waves will be described hereinafter.

The waves used for measuring scattered waves as used herein are, for example, ultrasonic waves, but may be radio waves such as microwaves, millimeter waves, or terahertz waves, or may be electromagnetic waves. The waves may also be light, or other sounds may be used.

The measurement area may be a region in the air, and the object may be a flying object. The object in the measurement area has a physical characteristic that is different from those of the surrounding media. Specifically, the physical characteristic is a physical characteristic that corresponds to the reflectance of the waves. In the case where radio waves are used as the waves, the physical characteristic may be the dielectric constant.

FIG. 11 is a block diagram illustrating a basic configuration of the imaging device according to the present embodiment. Imaging device 100 shown in FIG. 11 includes one or more transmitter arrays 111, one or more receiver arrays 112, and information processing circuit 130. Imaging device 100 may further include display 140.

Each transmitter array 111 is a circuit that transmits waves, and includes a plurality of transmitters 101 as circuit elements. Each transmitter 101 may be a transmitting element that transmits ultrasonic waves, such as a piezoelectric element. The plurality of transmitters 101 are connected in a first direction, and operate in conjunction with each other.

Transmitter array 111 transmits waves by the plurality of transmitters 101. More specifically, each transmitter 101 included in transmitter array 111 transmits waves. Transmitter array 111 transmits a wave formed by a plurality of waves transmitted by a plurality of transmitters 101 included in transmitter array 111.

Each receiver array 112 is a circuit that receives scattered waves, and includes a plurality of receivers 102 as circuit elements. Each receiver 102 may be a receiving element that receives ultrasonic waves, such as a piezoelectric element. The plurality of receivers 102 are connected in a second direction different from the first direction, and operate in conjunction with each other.

Receiver array 112 receives scattered waves by the plurality of receivers 102. More specifically, each receiver 102 included in receiver array 112 receives scattered waves. Receiver array 112 receives a scattered wave formed by a plurality of scattered waves received by a plurality of receivers 102 included in receiver array 112.

Information processing circuit 130 is a circuit that performs information processing. More specifically, information processing circuit 130 obtains measurement data of scattered waves, and images an object in the measurement area using the measurement data of the scattered waves. For example, information processing circuit 130 may perform computational processing indicated by the theory described above when imaging the object using the measurement data.

Information processing circuit 130 may also be a computer or a processor of a computer. Information processing circuit 130 may perform information processing by reading out a program from memory and executing the program. Alternatively, information processing circuit 130 may be a dedicated circuit that images an object in the measurement area in accordance with measurement data.

Information processing circuit 130 may be capable of communicating with the one or more transmitter arrays 111 and the one or more receiver arrays 112. Information processing circuit 130 may control the operation of the one or more transmitter arrays 111 and the one or more receiver arrays 112. Information processing circuit 130 may obtain position information and measurement data from the one or more transmitter arrays 111 and the one or more receiver arrays 112.

Information processing circuit 130 may be capable of communicating with transmission signal switcher 103 connected to the plurality of transmitter arrays 111. Information processing circuit 130 may control the operation whereby each transmitter array 111 transmits a wave by controlling transmission signal switcher 103. Alternatively, information processing circuit 130 may be capable of communicating with an actuator that moves transmitter array 111. Information processing circuit 130 may control the position of transmitter array 111 by controlling the actuator.

Information processing circuit 130 may be capable of communicating with reception signal switcher 104 connected to the plurality of receiver arrays 112. Information processing circuit 130 may control the operation whereby each receiver array 112 receives a wave by controlling reception signal switcher 104. Alternatively, information processing circuit 130 may be capable of communicating with an actuator that moves receiver array 112. Information processing circuit 130 may control the position of receiver array 112 by controlling the actuator.

In order to image the object, information processing circuit 130 may generate an image that indicates the object. Information processing circuit 130 may output the image indicating the object on display 140 or the like. Alternatively, information processing circuit 130 may output the image indicating the object to a printer (not illustrated in the drawings). As another alternative, information processing circuit 130 may transmit the image as electronic data to a different device (not illustrated in the drawings) via wired or wireless communication.

Display 140 is a display device such as a liquid crystal display. Note that display 140 is merely an arbitrary element and is not an essential element. Display 140 may be an external device that is not included in the configuration of imaging device 100.

FIG. 12 is a flowchart showing basic operations of imaging device 100 shown in FIG. 11. More specifically, the operations shown in FIG. 12 are performed by the elements of imaging device 100 shown in FIG. 11 such as the one or more transmitter arrays 111, the one or more receiver arrays 112, and information processing circuit 130.

First, each transmitter array 111 transmits waves to the measurement area via a plurality of transmitters 101 that are connected and operate in conjunction with each other in a first direction (S101). Each receiver array 112 receives waves from the measurement area via a plurality of receivers 102 that are connected and operate in conjunction with each other in a second direction different from the first direction (S102). Then, information processing circuit 130 images the object in the measurement area using measurement data of the scattered waves (S103, S104, and S105).

Specifically, information processing circuit 130 derives the scattering field function using the measurement data (S103). The scattering field function is a function that receives the transmission position of the wave and the reception position of the scattered wave as input and outputs the amount of the scattered wave at the reception position.

Next, information processing circuit 130 derives the imaging function using the scattering field function (S104). The imaging function is a function that receives an imaging target position as input and outputs an image intensity at the imaging target position, and is a function defined using an amount output from the scattering field function in response to inputting the imaging target position into the scattering field function as the transmission and reception positions.

Then, information processing circuit 130 images the object in the measurement area using the imaging function (S105).

This makes it possible to obtain measurement data corresponding to two directions using a plurality of transmitters 101 that transmit waves in coordination with each other, and a plurality of receivers 102 that receive scattered waves of the waves in coordination with each other. This makes it possible to accurately derive the scattering field function and the imaging function using the measurement data corresponding to two directions. This in turn makes it possible to image the object in the measurement area efficiently.

For example, each of the one or more transmitter arrays 111 and the one or more receiver arrays 112 may be a phased array. This makes it possible to transmit a wave in an adaptively determined direction and to receive a scattered wave from an adaptively determined direction. This in turn makes it possible to efficiently image the object in an adaptively determined range.

For example, the one or more transmitter arrays 111 may be one transmitter array 111 that moves in the second direction, and the one or more receiver arrays 112 may be one receiver array 112 that moves in the first direction. This makes it possible to reduce the plurality of transmitter arrays 111 and the plurality of receiver arrays 112. Therefore, it becomes possible to reduce the cost for preparing and arranging the plurality of transmitter arrays 111 and the plurality of receiver arrays 112.

For example, the one or more transmitter arrays 111 may be a plurality of transmitter arrays 111 arranged in the second direction, and the one or more receiver arrays 112 may be a plurality of receiver arrays 112 arranged in the first direction. The plurality of transmitter arrays 111 may sequentially transmit waves. This makes it possible to reduce the mechanism for moving transmitter array 111 and receiver array 112. Moreover, it becomes possible to reduce the time required for moving transmitter array 111 and receiver array 112 during measurement.

Also, for example, the one or more transmitter arrays 111 and the one or more receiver arrays 112 may be arranged on a flat surface. This makes it possible to simplify the arrangement of the one or more transmitter arrays 111 and the one or more receiver arrays 112. Therefore, it becomes possible to reduce the cost for arranging the one or more transmitter arrays 111 and the one or more receiver arrays 112. It also becomes possible to reduce the amount of computation required for deriving the scattering field function and imaging function.

Also, for example, the one or more transmitter arrays 111 and the one or more receiver arrays 112 may be arranged on a curved surface. This makes it possible to arrange the one or more transmitter arrays 111 and the one or more receiver arrays 112 on a curved surface according to the characteristics of the one or more transmitter arrays 111, the characteristics of the one or more receiver arrays 112, or the characteristics of the measurement area. This makes it possible to flexibly arrange the one or more transmitter arrays 111 and the one or more receiver arrays 112.

Also, for example, the one or more transmitter arrays 111 and the one or more receiver arrays 112 may be arranged on a flat surface. The first direction may be the y-axis direction. The second direction may be the x-axis direction.

The scattering field function may be expressed as follows.

[Math. 75]

$$\Phi(x_1, y_1, z, \alpha, \beta, k) = \frac{1}{(2\pi)^2} \int_{-\infty}^{\infty} \int_{-\infty}^{\infty} e^{-i(k_{x_1}x_1 + k_{y_1}y_1)} \tilde{Q}(k_{x_1}, k_{y_1}, \alpha, \beta, k)$$

$$\cdot e^{iz\left\{\sqrt{k^2 - \left\{k_{x_1}^2 + \beta^2 k^2\right\}} \pm \sqrt{k^2 - \left\{k_{y_1}^2 + \alpha^2 k^2\right\}}\right\}} dk_{x_1} dk_{y_1}$$

Here, z of the scattering field function represents the z-coordinate of the transmission position and reception position. $x_1$ of the scattering field function represents the x-coordinate of the transmission position. $y_1$ of the scattering field function represents the y-coordinate of the reception position. k represents the wavenumber of the wave. $k_{x1}$ and $k_{y1}$ represent the wavenumbers with respect to $x_1$ and $y_1$ of the scattering field function. $\alpha$ represents the phase factor of the one or more receiver arrays 112. $\beta$ represents the phase factor of the one or more transmitter arrays 111.

Also,

[Math. 76]

$$\tilde{Q}$$

represents the measurement data that has been Fourier transformed.

This makes it possible to accurately derive the scattering field function according to the arrangement of the one or more transmitter arrays 111 and the one or more receiver arrays 112 on a flat surface. Therefore, it becomes possible to accurately derive the scattering field function corresponding to the arrangement of the one or more transmitter arrays 111 and the one or more receiver arrays 112.

For example, the imaging function may be expressed as follows.

[Math. 77]

$$\rho(x, y, z)$$

$$= \frac{1}{(2\pi)^2} \int_0^{\infty} \int_{-\infty}^{\infty} \int_{-\infty}^{\infty} e^{-i(k_{x_1}x + k_{y_1}y)} \sum_{\alpha} \sum_{\beta} \left\{ \tilde{Q}(k_{x_1}, k_{y_1}, \alpha, \beta, k) \cdot e^{ik_z z} \right\} dk \, dk_{x_1} dk_{y_1}$$

Here, x, y, and z of the imaging function represent the x-coordinate, y-coordinate, and z-coordinate of the imaging target position. $k_z$ is defined as follows.

[Math. 78]

$$k_z = \sqrt{k^2 - \left\{k_{x_1}^2 + \beta^2 k^2\right\}} \pm \sqrt{k^2 - \left\{k_{y_1}^2 + \alpha^2 k^2\right\}}$$

This makes it possible to accurately derive the imaging function corresponding to the scattering field function formulated according to the arrangement of the one or more transmitter arrays 111 and the one or more receiver arrays 112 on a flat surface.

For example, the imaging function may be expressed as follows.

[Math. 79]

$$\rho(x,y,z)$$
$$= \frac{1}{(2\pi)^2} \int_0^\infty \int_{-\infty}^\infty \int_{-\infty}^\infty e^{-i(k_{x_1}x+k_{y_1}y)} \sum_\alpha \sum_\beta \left\{ \tilde{Q}(k_{x_1},k_{y_1},\alpha,\beta,k) \cdot e^{ik_zz} \right\} \left( \frac{dk}{dk_z} \right) dk_{x_1} dk_{y_1} dk_z$$

Here, x, y, and z of the imaging function represent the x-coordinate, y-coordinate, and z-coordinate of the imaging target position. $k_z$ and $dk/dk_z$ are defined as follows.

[Math. 80]

$$k_z = \sqrt{k^2 - \left\{ k_{x_1}^2 + \beta^2 k^2 \right\}} \pm \sqrt{k^2 - \left\{ k_{y_1}^2 + \alpha^2 k^2 \right\}}$$

$$\frac{dk}{dk_z} = \frac{1}{\dfrac{(1-\beta^2)k}{\sqrt{(1-\beta^2)k^2 - k_{x_1}^2}} + \dfrac{(1-\alpha^2)k}{\sqrt{(1-\alpha^2)k^2 - k_{y_1}^2}}}$$

This makes it possible to accurately derive the imaging function corresponding to the scattering field function formulated according to the arrangement of the one or more transmitter arrays 111 and the one or more receiver arrays 112 on a flat surface. This also makes it possible to perform calculations efficiently according to the transformed integration variables.

For example, the one or more transmitter arrays 111 and the one or more receiver arrays 112 may be arranged on a curved surface. The first direction may be the y-axis direction. The second direction may be the x-axis direction.

The scattering field function may be expressed as follows.

[Math. 81]

$$\Phi(x_1,y_1,z,k)$$
$$= \frac{1}{(2\pi)^4} \int_{-\infty}^\infty \int_{-\infty}^\infty \int_{-\infty}^\infty \int_{-\infty}^\infty e^{-i(k_{x_1}x_1+k_{y_1}y_1)} \left\{ \sum_{x_2,y_2} e^{-i(k_{x_2}x_2+k_{y_2}y_2)} e^{iv_2\sqrt{k^2-k_{x_1}^2-k_{y_2}^2}} e^{iu_2\sqrt{k^2-k_{y_1}^2-k_{x_2}^2}} \right\}$$
$$\cdot b(k_{x_1},k_{y_1},k) e^{iz\left(\sqrt{k^2-k_{x_1}^2-k_{y_2}^2}+\sqrt{k^2-k_{y_1}^2-k_{x_2}^2}\right)} dk_{x_1} dk_{y_1} dk_{x_2} dk_{y_2}$$

Here, z of the scattering field function represents the z-coordinate of the transmission position and reception position. $x_1$ of the scattering field function represents the x-coordinate of the transmission position. $y_1$ of the scattering field function represents the y-coordinate of the reception position. k represents the wavenumber of the wave. $k_{x1}$, $k_{y1}$, $k_{x2}$, and $k_{y2}$ represent integration variables.

b is defined as follows.

[Math. 82]

$$b(k_{x_1},k_{y_1},k) = \sum_{I,J} b_{I,J}(k_{x_1},k_{y_1},k)$$

I represents the index of the x-coordinate at which the one or more transmitter arrays 111 transmit the wave. J represents the index of the y-coordinate at which the one or more receiver arrays 112 receive the scattered wave.

$b_{I,J}$ is defined as follows.

[Math. 83]

$$b_{I,J}\left(k_{x_1},k_{y_1},k\right)=\frac{e^{i(k_{x_1}x_I+k_{y_1}y_J)}\Phi(x_I,y_J,0,k)}{\dfrac{1}{(2\pi)^2}\displaystyle\int_{-\infty}^{\infty}\int_{-\infty}^{\infty}G_{I,J}\left(k_{x_1},k_{y_1},k_{x_2},k_{y_2},k\right)dk_{x_2}dk_{y_2}}$$

Here, $\Phi(x_I, y_J, 0, k)$ represents the measurement data.

$G_{I,J}$ is defined as follows.

[Math. 84]

$$G_{I,J}\left(k_{x_1},k_{y_1},k_{x_2},k_{y_2},k\right)$$
$$=\sum_{m=-\infty}^{\infty}\sum_{n=-\infty}^{\infty}e^{-i(k_{x_2}m\Delta x+k_{y_2}m\Delta y)}e^{iv_2(x_I,n\Delta y)\sqrt{k^2-k_{x_1}^{\,2}-k_{y_2}^{\,2}}}e^{iu_2(y_J,m\Delta x)\sqrt{k^2-k_{y_1}^{\,2}-k_{x_2}^{\,2}}}$$

$\Delta x$ represents the distance between two adjacent receivers 102 among the plurality of receivers 102. $\Delta y$ represents the distance between two adjacent transmitters 101 among the plurality of transmitters 101. Each of $v_2$ and $u_2$ represents a function expressing the shape of the curved surface.

This makes it possible to accurately derive the scattering field function according to the arrangement of the one or more transmitter arrays 111 and the one or more receiver arrays 112 on a curved surface. Therefore, it becomes possible to accurately derive the scattering field function corresponding to the arrangement of the one or more transmitter arrays 111 and the one or more receiver arrays 112.

For example, the imaging function may be expressed as follows.

[Math. 85]

$$\rho(x,y,z)=\int_0^{\infty}\Phi(x,y,z,k)dk$$

Here, x, y, and z of the imaging function represent the x-coordinate, y-coordinate, and z-coordinate of the imaging target position.

This makes it possible to accurately derive the imaging function corresponding to the scattering field function formulated according to the arrangement of the one or more transmitter arrays 111 and the one or more receiver arrays 112 on a flat surface.

For example, other configurations, processes, and mathematical expressions described in the present embodiment are applicable as appropriate to the configurations, processes, and mathematical expressions described above as to the basic configuration and the basic operations. The equations and so on given in the present embodiment may be modified and applied as appropriate. For example, it is possible to use a mathematical expression that represents substantially the same content as that given by the mathematical expression described above, or to use any other mathematical expression derived based on the theory described above.

(Additional Comments)

While some aspects of the imaging device have been described thus far with reference to the embodiment, the modes of the imaging device are not limited to this embodiment. Any modification conceivable by those skilled in the art may be made to the embodiment, and a plurality of elements according to the embodiment may be combined arbitrarily. For example, processing that is executed by a specific element according to the embodiment may be executed by a different element, instead of the specific element. Moreover, a sequence of a plurality of processes may be changed, or a plurality of processes may be executed in parallel.

The imaging method including steps executed by each element of the imaging device may be executed by any arbitrary device or system. For example, part or all of the imaging method may be executed by a computer that includes, for example, a processor, memory, and an input/output circuit. At this time, a program for causing the computer to execute the imaging method may be executed by the computer to execute the imaging method.

The above-described program may be recorded on a non-transitory computer-readable recording medium.

Each element of the imaging device may be configured by dedicated hardware or by general-purpose hardware that executes the above-described program or the like, or may be configured by a combination of them. The general-purpose hardware may be configured by, for example, memory that records the program and a general-purpose processor that reads out and executes the program from the memory. The memory as used herein may, for example, be semiconductor memory or a hard disk, and the general-purpose processor may, for example, be a CPU.

The dedicated hardware may be configured by, for example, memory and a dedicated processor. For example, the dedicated processor may execute the imaging method described above with reference to the memory for recording measurement data.

Each element of the imaging device may be an electrical circuit. These electrical circuits may be configured as a single electrical circuit as a whole, or each may be a different electrical circuit. These electrical circuits may correspond to dedicated hardware or general-purpose hardware that executes the above-described program or the like.

The imaging device is not limited to being a physically integrated device, and may include a plurality of sub-devices arranged in a distributed manner. The imaging device may also be referred to as an imaging system.

[Industrial Applicability]

One aspect of the present disclosure is useful in imaging devices that image objects in a measurement area using measurement data of scattered waves, and is applicable in ultrasonic diagnostic devices and metal detection systems or the like.

[Reference Signs List]

100    imaging device
101    transmitter
102    receiver
103    transmission signal switcher
104    reception signal switcher
111    transmitter array
112    receiver array
130    information processing circuit
140    display
150    two-dimensional transceiver element array

**Claims**

1.  An imaging device comprising:

    one or more transmitter arrays each of which (i) includes a plurality of transmitters that are connected in a first direction and operate in conjunction with each other and (ii) transmits a wave to a measurement area via the plurality of transmitters;
    one or more receiver arrays each of which (i) includes a plurality of receivers that are connected in a second direction different from the first direction and operate in conjunction with each other and (ii) receives a scattered wave of the wave from the measurement area via the plurality of receivers; and
    an information processing circuit that images an object in the measurement area using measurement data of the scattered wave, wherein
    in imaging the object, the information processing circuit:

    derives, using the measurement data, a scattering field function that receives a transmission position of the wave and a reception position of the scattered wave as input and outputs an amount of the scattered wave at the reception position;
    derives an imaging function that receives an imaging target position as input and outputs an image intensity at the imaging target position, and is defined using an amount output from the scattering field function in response to inputting the imaging target position into the scattering field function as the transmission position and the reception position; and
images the object in the measurement area using the imaging function.

2. The imaging device according to claim 1, wherein
each of the one or more transmitter arrays and the one or more receiver arrays is a phased array.

3. The imaging device according to claim 1 or 2, wherein

the one or more transmitter arrays is one transmitter array that moves in the second direction, and
the one or more receiver arrays is one receiver array that moves in the first direction.

4. The imaging device according to claim 1 or 2, wherein

the one or more transmitter arrays comprises a plurality of transmitter arrays arranged in the second direction,
the one or more receiver arrays comprises a plurality of receiver arrays arranged in the first direction, and
the plurality of transmitter arrays sequentially transmit the wave.

5. The imaging device according to claim 1 or 2, wherein
the one or more transmitter arrays and the one or more receiver arrays are arranged on a flat surface.

6. The imaging device according to claim 1 or 2, wherein
the one or more transmitter arrays and the one or more receiver arrays are arranged on a curved surface.

7. The imaging device according to claim 2, wherein

the one or more transmitter arrays and the one or more receiver arrays are arranged on a flat surface,
the first direction is a y-axis direction,
the second direction is an x-axis direction,
the scattering field function is expressed as:

[Math. 1]

$$\Phi(x_1,y_1,z,\alpha,\beta,k)=\frac{1}{(2\pi)^2}\int_{-\infty}^{\infty}\int_{-\infty}^{\infty}e^{-i(k_{x_1}x_1+k_{y_1}y_1)}\tilde{Q}(k_{x_1},k_{y_1},\alpha,\beta,k)$$
$$\cdot e^{iz\left\{\sqrt{k^2-\left\{k_{x_1}{}^2+\beta^2k^2\right\}}\pm\sqrt{k^2-\left\{k_{y_1}{}^2+\alpha^2k^2\right\}}\right\}}dk_{x_1}dk_{y_1}$$

where z of the scattering field function represents a z-coordinate of the transmission position and the reception position, $x_1$ of the scattering field function represents an x-coordinate of the transmission position, $y_1$ of the scattering field function represents a y-coordinate of the reception position, k represents a wavenumber of the wave, $k_{x1}$ and $k_{y1}$ represent wavenumbers with respect to $x_1$ and $y_1$ of the scattering field function, $\alpha$ represents a phase factor of the one or more receiver arrays, and $\beta$ represents a phase factor of the one or more transmitter arrays, and

[Math. 2]

$$\tilde{Q}$$

represents the measurement data that has been Fourier transformed.

8. The imaging device according to claim 7, wherein

the imaging function is expressed as:

[Math. 3]

$$\rho(x,y,z)$$

$$= \frac{1}{(2\pi)^2} \int_0^\infty \int_{-\infty}^\infty \int_{-\infty}^\infty e^{-i(k_{x_1}x+k_{y_1}y)} \sum_\alpha \sum_\beta \left\{ \tilde{Q}(k_{x_1}, k_{y_1}, \alpha, \beta, k) \cdot e^{ik_z z} \right\} dk \, dk_{x_1} dk_{y_1}$$

where x, y, and z of the imaging function respectively represent an x-coordinate, a y-coordinate, and a z-coordinate of the imaging target position, and
$k_z$ is defined as:

[Math. 4]

$$k_z = \sqrt{k^2 - \left\{ k_{x_1}^2 + \beta^2 k^2 \right\}} \pm \sqrt{k^2 - \left\{ k_{y_1}^2 + \alpha^2 k^2 \right\}}$$

9.  The imaging device according to claim 7, wherein

the imaging function is expressed as:

[Math. 5]

$$\rho(x,y,z)$$

$$= \frac{1}{(2\pi)^2} \int_0^\infty \int_{-\infty}^\infty \int_{-\infty}^\infty e^{-i(k_{x_1}x+k_{y_1}y)} \sum_\alpha \sum_\beta \left\{ \tilde{Q}(k_{x_1}, k_{y_1}, \alpha, \beta, k) \cdot e^{ik_z z} \right\} \left( \frac{dk}{dk_z} \right) dk_{x_1} dk_{y_1} dk_z$$

where x, y, and z of the imaging function respectively represent an x-coordinate, a y-coordinate, and a z-coordinate of the imaging target position, and
$k_z$ and $dk/dk_z$ are defined as:

[Math. 6]

$$k_z = \sqrt{k^2 - \left\{ k_{x_1}^2 + \beta^2 k^2 \right\}} \pm \sqrt{k^2 - \left\{ k_{y_1}^2 + \alpha^2 k^2 \right\}}$$

$$\frac{dk}{dk_z} = \frac{1}{\dfrac{(1-\beta^2)k}{\sqrt{(1-\beta^2)k^2 - k_{x_1}^2}} + \dfrac{(1-\alpha^2)k}{\sqrt{(1-\alpha^2)k^2 - k_{y_1}^2}}}$$

.

10. The imaging device according to claim 1, wherein

the one or more transmitter arrays and the one or more receiver arrays are arranged on a curved surface,
the first direction is a y-axis direction,
the second direction is an x-axis direction,
the scattering field function is expressed as:

[Math. 7]

$$\Phi(x_1, y_1, z, k)$$

$$= \frac{1}{(2\pi)^4} \int_{-\infty}^{\infty}\int_{-\infty}^{\infty}\int_{-\infty}^{\infty}\int_{-\infty}^{\infty} e^{-i(k_{x_1}x_1+k_{y_1}y_1)} \left\{ \sum_{x_2,y_2} e^{-i(k_{x_2}x_2+k_{y_2}y_2)} e^{iv_2\sqrt{k^2-k_{x_1}^2-k_{y_2}^2}} e^{iu_2\sqrt{k^2-k_{y_1}^2-k_{x_2}^2}} \right\}$$

$$\cdot b(k_{x_1}, k_{y_1}, k) e^{iz\left(\sqrt{k^2-k_{x_1}^2-k_{y_2}^2}+\sqrt{k^2-k_{y_1}^2-k_{x_2}^2}\right)} dk_{x_1} dk_{y_1} dk_{x_2} dk_{y_2}$$

where z of the scattering field function represents a z-coordinate of the transmission position and the reception position, $x_1$ of the scattering field function represents an x-coordinate of the transmission position, $y_1$ of the scattering field function represents a y-coordinate of the reception position, k represents a wavenumber of the wave, and $k_{x1}$, $k_{y1}$, $k_{x2}$, and $k_{y2}$ represent integration variables,
b is defined as:

[Math. 8]

$$b(k_{x_1}, k_{y_1}, k) = \sum_{I,J} b_{I,J}(k_{x_1}, k_{y_1}, k)$$

where I represents an index of an x-coordinate at which the one or more transmitter arrays transmit the wave, and J represents an index of a y-coordinate at which the one or more receiver arrays receive the scattered wave, $b_{I,J}$ is defined as:

[Math. 9]

$$b_{I,J}(k_{x_1}, k_{y_1}, k) = \frac{e^{i(k_{x_1}x_I+k_{y_1}y_J)}\Phi(x_I, y_J, 0, k)}{\dfrac{1}{(2\pi)^2}\int_{-\infty}^{\infty}\int_{-\infty}^{\infty} G_{I,J}(k_{x_1}, k_{y_1}, k_{x_2}, k_{y_2}, k)\, dk_{x_2} dk_{y_2}}$$

where $\Phi$ represents the measurement data, and $G_{I,J}$ is defined as:

[Math. 10]

$$G_{I,J}(k_{x_1}, k_{y_1}, k_{x_2}, k_{y_2}, k)$$

$$= \sum_{m=-\infty}^{\infty}\sum_{n=-\infty}^{\infty} e^{-i(k_{x_2}m\Delta x+k_{y_2}n\Delta y)} e^{iv_2(x_I, n\Delta y)\sqrt{k^2-k_{x_1}^2-k_{y_2}^2}} e^{iu_2(y_J, m\Delta x)\sqrt{k^2-k_{y_1}^2-k_{x_2}^2}}$$

where $\Delta x$ represents a distance between two adjacent receivers among the plurality of receivers, $\Delta y$ represents a distance between two adjacent transmitters among the plurality of transmitters, and each of $v_2$ and $u_2$ represents a function expressing a shape of the curved surface.

11. The imaging device according to claim 10, wherein

the imaging function is expressed as:

[Math. 11]

$$\rho(x, y, z) = \int\limits_{0}^{\infty} \Phi(x, y, z, k)dk$$

where x, y, and z of the imaging function respectively represent an x-coordinate, a y-coordinate, and a z-coordinate of the imaging target position.

12. An imaging method comprising:

transmitting a wave to a measurement area via a plurality of transmitters included in one or more transmitter arrays in each of which the plurality of transmitters are connected in a first direction and operate in conjunction with each other;
receiving a scattered wave of the wave from the measurement area via a plurality of receivers included in one or more receiver arrays in each of which the plurality of receivers are connected in a second direction different from the first direction and operate in conjunction with each other; and
imaging an object in the measurement area using measurement data of the scattered wave, wherein
the imaging of the object includes:

deriving, using the measurement data, a scattering field function that receives a transmission position of the wave and a reception position of the scattered wave as input and outputs an amount of the scattered wave at the reception position;
deriving an imaging function that receives an imaging target position as input and outputs an image intensity at the imaging target position, and is defined using an amount output from the scattering field function in response to inputting the imaging target position into the scattering field function as the transmission position and the reception position; and
imaging the object in the measurement area using the imaging function.

FIG. 1

FIG. 2

One-dimensional array

Linear scanning

# FIG. 3

EP 4 678 117 A1

# FIG. 4

111

Delay

101

Δt5

Δt4

Δt3

Δt2

Δt1

# FIG. 5

112

Delay

102

Aggregation

Δt5

Δt4

Δt3

Δt2

Δt1

# FIG. 6

# FIG. 7

# FIG. 8

Scanning in y-axis direction (y1)

$(x, y, z)$

102   $\Delta x$   $P_2(x_2, y_1, z)$   112

101

$\Delta y$

111

Scanning in x-axis direction (x1)

$P_1(x_1, y_2, z)$

Transmission wave

$\rho_1$

Phase difference: $\beta k \Delta y$

$\rho_2$

Reception wave

Phase difference: $\alpha k \Delta x$

$P(\xi, \eta, \zeta)$

x

z

y

EP 4 678 117 A1

# FIG. 9

150

FIG. 10

# FIG. 11

100

111 Transmitter array
101 Transmitter

112 Receiver array
102 Receiver

130 Information processing circuit

140 Display

# FIG. 12

START

Transmit waves to
measurement area — S101

Receive scattered waves
from measurement area — S102

Derive scattering
field function — S103

Derive imaging function using
scattering field function — S104

Image object in measurement
area using imaging function — S105

END

## INTERNATIONAL SEARCH REPORT

| International application No. |
|---|
| **PCT/JP2024/003831** |

**A. CLASSIFICATION OF SUBJECT MATTER**

*A61B 8/13*(2006.01)i; *G01N 29/06*(2006.01)i; *G01S 7/02*(2006.01)i; *G01S 13/89*(2006.01)i; *G01S 15/89*(2006.01)i
FI: A61B8/13; G01S13/89; G01S7/02 216; G01N29/06; G01S15/89 B

According to International Patent Classification (IPC) or to both national classification and IPC

**B. FIELDS SEARCHED**

Minimum documentation searched (classification system followed by classification symbols)

A61B8/00-8/15; G01N29/00-29/52; G01S1/72-15/96

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | WO 2021/053971 A1 (INTEGRAL GEOMETRY SCIENCE INC.) 25 March 2021 (2021-03-25) <br> entire text, all drawings | 1-12 |
| A | WO 2021/020387 A1 (INTEGRAL GEOMETRY SCIENCE INC.) 04 February 2021 (2021-02-04) <br> entire text, all drawings | 1-12 |
| A | WO 2022/265017 A1 (KIMURA, Kenjiro) 22 December 2022 (2022-12-22) <br> entire text, all drawings | 1-12 |
| A | US 2013/0096433 A1 (SONETICS ULTRASOUND, INC.) 18 April 2013 (2013-04-18) <br> entire text, all drawings | 1-12 |

☐ Further documents are listed in the continuation of Box C.    ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **15 April 2024** | **23 April 2024** |

| Name and mailing address of the ISA/JP | Authorized officer |
|---|---|
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (July 2022)

**EP 4 678 117 A1**

| Patent document cited in search report | | | Publication date (day/month/year) | Patent family member(s) | | | Publication date (day/month/year) |
|---|---|---|---|---|---|---|---|
| WO | 2021/053971 | A1 | 25 March 2021 | US | 2022/0319067 | A1 | |
| | | | | entire text, all drawings | | | |
| | | | | EP | 4033228 | A4 | |
| | | | | CN | 114390908 | A | |
| | | | | KR | 10-2022-0062270 | A | |
| | | | | AU | 2020348001 | A1 | |
| | | | | CA | 3153233 | A1 | |
| | | | | IL | 290774 | A | |
| | | | | TW | 202115738 | A | |
| WO | 2021/020387 | A1 | 04 February 2021 | US | 2022/0268711 | A1 | |
| | | | | entire text, all drawings | | | |
| | | | | EP | 4009040 | A4 | |
| | | | | CN | 114144108 | A | |
| | | | | KR | 10-2022-0041088 | A | |
| | | | | AU | 2020323417 | A1 | |
| | | | | CA | 3148045 | A1 | |
| | | | | IL | 289641 | A | |
| | | | | TW | 202111572 | A | |
| WO | 2022/265017 | A1 | 22 December 2022 | AU | 2022292324 | A1 | |
| | | | | CA | 3222714 | A1 | |
| | | | | TW | 202305400 | A | |
| US | 2013/0096433 | A1 | 18 April 2013 | (Family: none) | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP S6266145 A
- WO 2014125815 A
- WO 2015136936 A
- WO 2021020387 A
- WO 2021053971 A
- WO 2022260112 A
- WO 2022265017 A